# EUROPEAN PATENT SPECIFICATION

(11) **EP 1 499 580 B1**
(45) Date of publication and mention of the grant of the patent: **28.03.2012**
(21) Application number: 03745384.2
(22) Date of filing: 31.03.2003
(51) Int. Cl.: C07C 69/757, C07C 233/63, C07C 217/08, A61K 31/215, A61K 31/164, A61K 31/13, A61P 3/08

(54) **COMPOUNDS THAT STIMULATE GLUCOSE UTILIZATION AND METHODS OF USE**
VERBINDUNGEN, DIE DIE GLUCOSENUTZUNG STIMULIEREN, UND ANWENDUNGSVERFAHREN
COMPOSES STIMULANT L'UTILISATION DU GLUCOSE ET PROCEDES D'UTILISATION

(30) Priority: 01.04.2002 WO PCT/IB02/02525; 01.04.2002 US 112041; 11.07.2002 US 181274; 05.12.2002 US 313990
(43) Date of publication of application: 26.01.2005
(73) Proprietor: The Governors of the University of Alberta, Edmonton, Alberta T6G 2E1 (CA)
(72) Inventor: LOPASCHUK, Gary, David, Edmonton, Alberta T6R 1H4 (CA); VEDERAS, John, Christopher, Edmonton, Alberta T6C 3X5 (CA); DYCK, Jason, Roland, Sherwood Park, Alberta T8G 1A4 (CA)
(74) Representative: Vossius & Partner
(86) International application number: PCT/IB2003/001761
(87) International publication number: WO 2003/082800

(56) References cited:
- WO-A-02/085294
- US-A- 3 306 727
- US-A- 3 957 849
- US-A- 4 000 315
- DATABASE CROSSFIRE BEILSTEIN [Online] Beilstein Institut zur Förderung der Chemischen Wissenschaften, Frankfurt am Main, DE; Database accession no. 2576757 XP002248454 & J. MED. CHEM., vol. 6, 1963, pages 221-227,
- DATABASE CROSSFIRE BEILSTEIN [Online] Beilstein Institut zur Förderung der Chemischen Wissenschaften, Frankfurt am Main, DE; Database accession no. 2040782 XP002248455 & J. MED. CHEM., vol. 39, no. 22, 1996, pages 4354-4357,
- DATABASE CROSSFIRE BEILSTEIN [Online] Beilstein Institut zur Förderung der Chemischen Wissenschaften, Frankfurt am Main, DE; Database accession no. 7079339 XP002248456 & J. MED. CHEM., vol. 37, no. 15, 1994, pages 2285-2291,
- BERSIN R M ET AL: "DICHLOROACETATE AS METABOLIC THERAPY FOR MYOCARDIAL ISCHEMIA AND FAILURE" AMERICAN HEART JOURNAL, MOSBY- YEAR BOOK INC., ST. LOUIS, MO, US, vol. 134, no. 5, PART 1, November 1997 (1997-11), pages 841-855, XP001032583 ISSN: 0002-8703 cited in the application
- DATABASE CA [Online] CHEMICAL ABSTRACTS SERVICE, COLUMBUS, OHIO, US; HENRICK, CLIVE A. ET AL: "Ovicidal activity and its relation to chemical structure for the two-spotted spider mite (Tetranychus urticae Koch) in a new class of miticides containing the cyclopropyl group" retrieved from STN Database accession no. 85:105335 CA XP002249230 & JOURNAL OF AGRICULTURAL AND FOOD CHEMISTRY (1976), 24(5), 1023-9 ,

## Description

### FIELD OF THE INTENTION

The invention relates to novel compounds that stimulate rates of glucose oxidation in myocardial cells. The invention also relates to pharmaceutical compositions comprising compounds capable of stimulating glucose oxidation, particulary in myocardial cells. The compounds are also capable of treating myocardial ischemia.

### BACKGROUND OF THE INVENTION

Myocardial ischemia is a common clinical pathology that occurs in the setting of angina pectoris, acute myocardial infarction, or during cardiac surgery. Myocardial ischemia is a major clinical problem, with its complications being the major cause of mortality and morbidity in Western society.

It has been reported that stimulating glucose oxidation both during and following ischemia can benefit the ischemic heart. Br J Pharmacol 128: 197-205, 1999, Am J Physiol 275: H1533-41, 1998. Biochimica et Biophysica Acta 1225: 191-9, 1994, Pediatric Research 34: 735-41, 1993, Journal of Biological Chemistry 270: 17513-20, 1995. Biochimica et Biophysica Acta 1301: 67-75, 1996, Am J Cardiol 80: 11A-16A, 1997, Molecular & Cellular Biochemistry 88: 175-9, 1989, Circ Res 65: 378-87, 1989, Circ Res 66: 546-53, 1990, American Journal of Physiology 259: H1079-85, 1990, American Journal of Physiology 261: H1053-9, 1991, Am J Physiol Heart Circ Physiol 280: H1762-9, 2001, J Am Coll Cardiol 36: 1378-85, 2000.

To meet the high energy demands of the contracting muscle, the heart must produce a constant and plentiful supply of the free energy carrier, adenosine triphosphate (ATP). This energy is produced by the metabolism of a variety of carbon substrates, including carbohydrates such as glucose. The metabolism of fatty acid is the other major source of energy for the heart.

Glucose metabolism in the heart consists of two important pathways, namely glycolysis and glucose oxidation.

It has been shown that during ischemia (such as that produced by angina pectoris, myocardial infarction or heart surgery) the levels of circulating fatty acids in the plasma can be dramatically elevated. Am Heart J 128: 61-7, 1994. As a result, during ischemia and reperfusion the heart is exposed to high levels of fatty acids, which results in the preferential use of fatty acids as an oxidative substrate over glucose. It further has been reported that this over-reliance on fatty acids as a major source of ATP contributes to fatty acid-induced ischemic damage. This observation has sparked numerous approaches directed at switching substrate utilization back to glucose in an attempt to protect the heart from fatty acid-induced ischemic damage. J Cardiovasc Pharmacol 31: 336-44, 1998, Am Heart J 134: 841-55, 1997, Am J Physiol 273: H2170-7, 1997, Cardiovasc Drugs Ther 14: 615-23, 2000, Cardiovasc Res 39: 381-92, 1998, Am Heart J 139: S115-9, 2000, Coron Artery Dis 12: S8-11, 2001, Am J Cardiol 82: 14K-17K, 1998, Molecular & Cellular Biochemistry 172: 137-47, 1997, Circulation 95: 313-5, 1997, Gen Pharmacol 30: 639-45, 1998, Am J Cardiol 82: 42K-49K, 1998, Coron Artery Dis 12: S29-33, 2001, Coron Artery Dis 12: S3-7, 2001, J Nucl Med 38: 1515-21, 1997. Current approaches that are used to manipulate myocardial energy metabolism involve either stimulating glucose metabolism directly or indirectly (i.e., inhibiting fatty acid metabolism).

Since high fatty acid oxidation rates markedly decrease glucose oxidation, one approach to increasing glucose oxidation is to inhibit fatty acid oxidation. This has proven effective both during and following ischemia, and this pharmacological approach is starting to see clinical use. Although a number of pharmacological agents designed to inhibit fatty acid oxidation have recently been developed, the direct β-oxidation inhibitor, trimetazidine, was the first anti-anginal agent widely used that has a mechanism of action that can be attributed to an optimization of energy metabolism Circulation Research. 86: 580-8, 2000 .

Trimetazidine is reported to primarily act by inhibiting fatty acid oxidation, thereby stimulating glucose oxidation in the heart.

A second clinically effective agent that is reported to switch energy metabolism from fatty acid to glucose oxidation is ranolazine. It has been reported that this agent stimulates glucose oxidation secondary to an inhibition of fatty acid oxidation Circulation 93: 135-42, 1996.

The detrimental effects of fatty acids on mechanical function during and following ischemia may also be attenuated by agents that increase glucose oxidation directly. Numerous experimental studies have reported that stimulation of glucose oxidation by using dichloroacetate (DCA) following ischemia (at the expense of fatty acids) can benefit the ischemic heart. Am Heart J 134: 841-55, 1997. Although DCA is an effective compound designed to stimulate glucose oxidation, it has a short biological half-life.

Therefore, there is need to develop novel class of compounds and to identify compounds that can stimulate glucose oxidation, have long biological life, and be effective in treatment or prevention of myocardial ischemia.

### SUMMARY OF THE INVENTION

The present invention relates to a compound selected from
cyclopropanecarboxylic acid, 2-[2-(2-methoxy-ethoxy)-ethoxy]-ethyl ester;
cyclobutanecarboxylic acid, 2-[2-(2-methoxy-ethoxy)-ethoxy]-ethyl ester;
cyclopropanecarboxylic acid, 2-(2-benzyloxy-ethoxy)-ethyl ester;
cyclobutanecarboxylic acid, 2-(2-benzyloxy-ethoxy)-ethyl ester;
cyclobutanecarboxylic acid, 2-(2-butoxy-ethoxy)-ethyl ester;
cyclobutanecarboxylic acid, 2-(2-ethoxy-ethoxy)-ethyl ester;
cyclopropanecarboxylic acid, 2-(2-dimethylamino-ethoxy)-ethyl ester;
cyclobutanecarboxylic acid, 2-(2-dimethylamino-ethoxy)-ethyl ester;
cyclopropanecarboxylic acid, 2-(2-hexyloxy-ethoxy)-ethyl ester;
cyclobutanecarboxylic acid, 2-(2-hexyloxy-ethoxy)-ethyl ester;
cyclopropanecarboxylic acid, 2-(2-methoxy-ethoxy)-ethyl ester;
cyclobutanecarboxylic acid, 2-(2-methoxy-ethoxy)-ethyl ester;
cyclopropanecarboxylic acid, 2-ethoxy-ethyl ester;
cyclobutanecarboxylic acid, 2-ethoxy-ethyl ester;
cyclopropanecarboxylic acid, 2-isopropoxy-ethyl ester; and
cyclobutanecarboxylic acid, 2-isopropoxy-ethyl ester;
or a pharmaceutically acceptable salt thereof.

In one embodiment the present invention refers to a pharmaceutical composition comprising at least one compound as defined above or a pharmaceutically acceptable salt thereof; and a pharmaceutically acceptable carrier, diluent, excipient or mixtures thereof. The pharmaceutical composition can be contained in a kit.

In one aspect the compounds of the present invention are suitable for increasing glucose utilization in a cell, tissue or organ of a warm blooded animal. In another aspect the compounds of the present invention are suitable for treating physiological conditions or disorders treatable by increasing glucose utilization such as ischemic/reperfusion injury, post myocardial infarction, angina, heart failure, a cardiomyopathy, peripheral vascular disease, diabetes, and lactic acidosis or symptoms or side effects associated with open heart surgery, bypass surgery or heart transplant.

Furthermore, compounds represented by Formula (I) are disclosed: 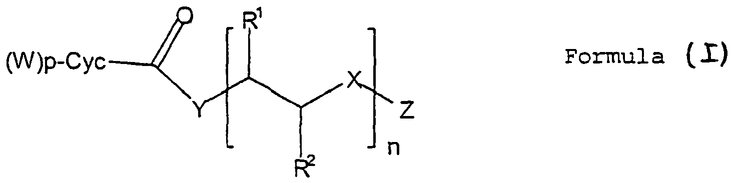 wherein
W is C₁-C₆ alkyl , halogen, or aryl;
Cyc is C₃ or C₄ cycloalkyl;
p is an integer from 0 to 3 when Cyc is C₄ cycloalkyl, and p is an integer from 0 to 2 when Cyc is C₃ cycloalkyl;
Y is O, S, or NR;
X is O, S, NR, or CR³R⁴;
Z is H, alkyl, cycloalkyl, aryl or (cyclo)alkylcarbonyl or 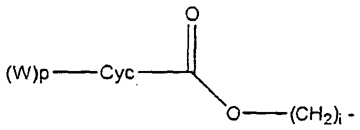
if X is NR and R is 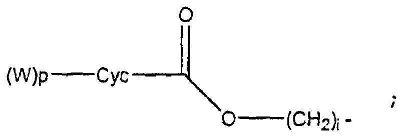
R is H, alkyl, aryl, or 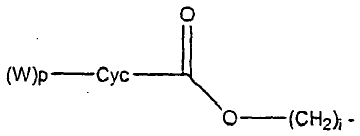
   where i is an integer from 2 to 4;
   R¹ is H, alkyl, aryl or O;
   R² is H, alkyl or aryl;
   R³ and R⁴ are independently H, alkyl or aryl; and n is an integer from 1 to 10; or a pharmaceutically acceptable salt thereof.

Compounds of Formula (I) which are represented by Formula (Ia) are disclosed: 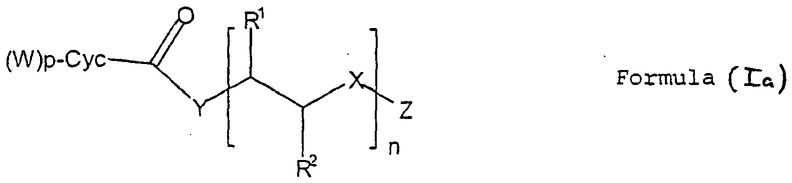 wherein
W is C₁-C₆ alkyl, halogen, or aryl;
Cyc is C₃ or C₄ cycloalkyl;
p is an integer from 0 to 3 when Cyc is C₄ cycloalkyl, and p is an integer from 0 to 2 when Cyc is C₃ cycloalkyl;
Y is O, S, or NR;
X is O, S, NR, or CR³R⁴
Z is H, alkyl, cycloalkyl, aryl or (cycle)alkylcarbonyl;
R is H, alkyl or acryl;
R¹ is H, alkyl, aryl or O;
R² is H, alkyl or aryl;
R³ and R⁴ are independently H, alkyl or aryl; and
n is an integer from 1 to 10;
or a pharmaceutically acceptable salt thereof.

According to one aspect, the compound of the present invention are suitable for increasing or improving glucose utilisation in myocardial or other types of cells, tissue or organs of warm blooded animals, especially those which are capable of high glucose metabolism (e.g., heart and other muscles).

According to an alternate aspect, the present invention is also directed to pharmaceutical compositions comprising a compound of the present invention and suitable pharmaceutical carriers, excipients or fillers.

According to a further aspect, the compounds of the present invention are also suitable for treating physiological conditions or disorders that may be effectively treated by increasing of cell glucose utilization.

The present invention is further directed to kits including a pharmaceutical composition according to the present invention.

The compounds of the present invention are applicable for treating warm blooded animal subjects, such as mammals, including humans, primates, etc.

### Definitions

As used herein, the term "alkyl" means straight or branched alkane chain, which may be, optionally substituted with, for example, halogens, cyclic or aromatic substituents.

As used herein, the terms "aryl" or "aromatic" refer to mono- and bi-cyclic structures comprising 5 to 12 carbon atoms, preferably monocyclic rings containing six carbon atoms. The ring may be optionally substituted with alkyl, alkenyl, halogen, alkoxy, or haloalkyl substituents.

### BRIEF DESCRIPTION OF THE DRAWINGS

Figure 1 is a graph which depicts glucose oxidation in an isolated perfused working rat heart model at the indicated concentrations of cyclopropanecarboxylic acid, 2-[2-(2- methoxy-ethoxy)-ethoxy]-ethyl ester (MM054) as compared to a control.
Figure 2 is a graph which depicts glucose oxidation in an isolated perfused working rat heart model at the indicated concentrations of cyclobutanecarboxylic acid, 2-[2-(2-methoxy-ethoxy)-ethoxy]-ethyl ester (MM056) as compared to a control.
Figure 3 is a graph which depicts glucose oxidation in an isolated perfused working rat heart model at increasing concentrations of cyclopropanecarboxylic acid, 2-isopropoxy-ethyl ester (MM070) as compared to a control.
Figure 4 (not according to the invention) is a graph which depicts glucose oxidation in an isolated perfused working rat heart model at increasing concentrations of cyclopropanecarboxylic acid (MM001) as compared to a control.

### DETAILED DESCRIPTION OF THE INVENTION

According to one aspect, the present invention provides novel compounds which are derivatives of a cyclopropane carboxylic acid or a cyclobutane carboxylic acid. These compounds exhibit glucose oxidation stimulating activity in myocardial cells and other types of cells. The compounds according to the present invention are as defined above. Formula (I) discloses further compounds. 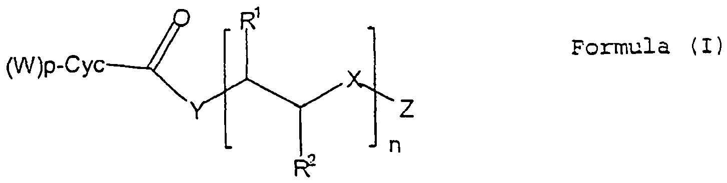 wherein
W is C₁-C₆ alkyl, halogen, or aryl;
Cyc is C₃ or C₄ cycloalkyl;
p is an integer from 0 to 3 when Cyc is C₄ cycloalkyl, and p is an integer from 0 to 2 when Cyc is C₃ cycloalkyl;
Y is O, S, or NR,
X is O, S, NR, or CR³R⁴;
Z is H, alkyl, cycloalkyl, aryl or (cyclo)alkyl carbonyl or 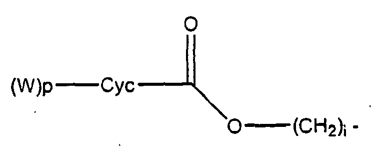
if X is NR and R is 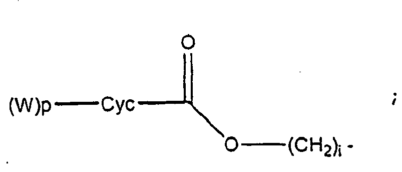
R is H, alkyl, aryl or 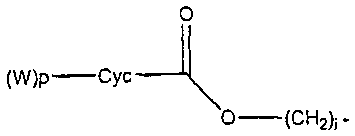
   where i is an integer from 2 to 4;
   R¹ is H, alkyl or aryl;
   R² is H, alkyl, aryl or O;
   R³ and R⁴ are independently H, alkyl or aryl and
   n is an integer from 1 to 10;
or a pharmaceutically acceptable salt thereof.

According to an alternate aspect compounds of Formula (I) which are represented by Formula (Ia) are disclosed : 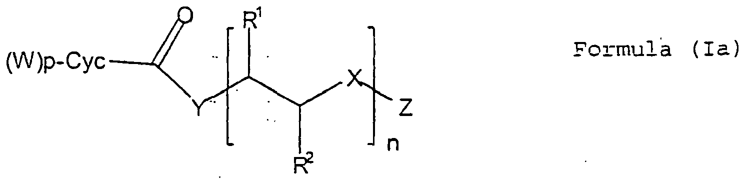 wherein
W is C₁-C₆ alkyl, halogen, or aryl;
Cyc is C₃ or C₄ cycloalkyl;
p is an integer from 0 to 3 when Cyc is C₄ cycloalkyl, and p is an integer from 0 to 2 when Cyc is C₃ cycloalkyl;
Y is O, S, or NR,
X is O, S, NR, or CR³R⁴;
Z is H, alkyl, cycloalkyl, aryl or (cyclo)alkyl carbonyl or
R is H, alkyl or aryl;
R¹ is H, alkyl or aryl;
R² is H, alkyl, aryl or O;
R³ and R⁴ are independently H, alkyl or aryl and
n is an integer from 1 to 10;
or a pharmaceutically acceptable salt thereof.

Certain compounds may be conveniently prepared from the appropriate substituted or unsubstituted cyclopropane carbonyl chloride or cyclobutane carbonyl chloride according to the following reaction scheme: 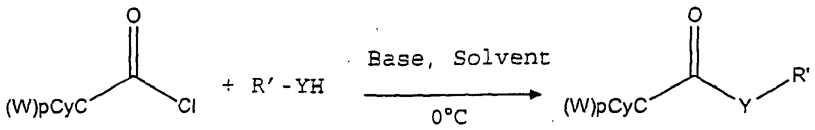
wherein W, Cyc, and p are as defined in connection with Formula (I), Y is O such that R' YH is an alcohol and
R' is 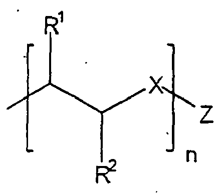 where R¹, R², X, Z and n are as defined in connection with Formula (Ia) .

Other compounds of Formula (I) may be prepared by methods similar to those described in Example 23 and using the appropriate starting materials.

Suitable solvents include inert organic solvents such as dichloromethane and suitable base catalysts include triethylamine and pyridine.

Reaction conditions may be varied depending on the starting materials and the desired end product. Optimization of the reaction conditions would be apparent for one of ordinary skill.

Certain compounds have unsubstituted cycloalkyl rings (p is 0).

According to a further disclosed embodiment Y is O, and X is NR or O, n is 1 to 4, p is 0, R¹, R² , R³ and R⁴ are hydrogen, and Z is lower alkyl, cycloalkyl or phenyl; or Y is NR, and X is O n is 1 or 2, p is 0, R¹, R², R³ and R⁴ are hydrogen, and Z is hydrogen

Further compounds wich are not according to the invention are the following:
(cyclobutanecarbonyl-amino)-acetic acid;
2-(cyclopropanecarbonyl-amino)-propionic acid;
cyclopropanecarboxylic acid, 2-(2-cyclopropanecarbonylcxy-ethoxy)-ethyl ester;
cyclobutanecarboxylic acid, 2-(2-cyclobutanecarbonyloxy-ethoxy)-ethyl ester;
cyclopropanecarboxylic acid, 2-[2-(2-cyclopropanecarbonyloxy-ethoxy)-ethoxyJ-ethyl ester; and
cyclobutanecarboxylic acid, 2-[2-(2-cyclobutaneca.rbonyloxv-ethoxy)-ethoxy]-ethyl ester.

The compounds are capable of increasing the rate of glucose oxidation and improving glucose utilization in myocardial and other cells, tissue or organs of humans and animals. It has been discovered that the compounds of the present invention , such as cyclopropanecarboxylic acid, 2-[2-(2-methoxy-ethoxy)-ethoxyl-ethyl ester and cyclobutanecarboxylic acid, 2-[2-(2-methoxy-ethoxy)-ethoxy]-ethyl ester can increase glucose utilization in myocardial and other types of cells, tissue or organs of warm blooded animals, including humans.

Compounds of Formula II have the structure: 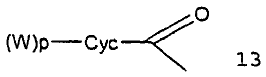 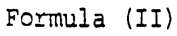 wherein
W is C₁-C₆ alkyl, halogen, or aryl;
Cyc is C₃ or C₄ cycloalkyl; and
p is an integer from 0 to 3 when Cyc is C₄ cycloalkyl, or p is an integer from 0 to 2 when Cyc is C₃ cycloalkyl.

Cells, tissue or organs of an animal are treated with at least one compound represented by Formula (I) or Formula (II) in an amount effective to stimulate glucose utilization. The compounds of the Formula (I) or Formula (II) may be delivered to the cells, tissues or organs by conventional means of administrating pharmaceutical compositions such as oral administration, injection or infusion, etc., of the compounds of the Formula (I) or (II) to the animal.

Also disclosed are pharmaceutical compositions comprising, as its active component, at least one compound according to the Formulas (I) or (II) or their pharmaceutically acceptable salts, esters or prodrugs. Pharmaceutical compositions comprising more than one compound according to the Formulas (I) or (II), their various mixtures and combinations are also contemplated.

Pharmaceutical compositions or formulations include compositions and formulations conventionally used in the pharmaceutical arts and may comprise carriers and excipients compatible with oral, intravenous, intramuscular, intraarterial, intracranial, and/or intracavity administration. Suitable pharmaceutical compositions and/or formulations may further compose colloidal dispersion systems, or lipid formulations (e.g., cationic or anionic lipids), micelles, microbeads, etc.

As noted, pharmaceutical compositions of the present invention may comprise pharmaceutically acceptable and physiologically acceptable carriers, diluents or excipients. Examples of suitable carriers, diluents and excipients include solvents (aqueous or non-aqueous), solutions, emulsions, dispersion media, coatings, isotonic and absorption promoting or delaying agents, compatible with pharmaceutical administration, and other commonly used carriers known in the art.

Pharmaceutical compositions may also include carriers to protect the composition against rapid degradation or elimination from the body, and, thus may comprise a controlled release formulation, including implants and microencapsulated delivery systems. For example, a time delay material such as glyceryl monostearate or glyceryl stearate alone, or in combination with a wax, may be employed.

Pharmaceutical compositions can be formulated to be compatible with a particular route of administration. For oral administration, a composition can be incorporated with excipients and used in the form of tablets, pills or capsules, e.g., gelatin capsules. Pharmaceutically compatibly binding agents, and/or adjuvant materials can be included in oral formulations. The tablets, pills, capsules, etc., can contain any of the following ingredients, or similar compounds: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; or a flavoring or sweetening agent.

Pharmaceutical compositions for parenteral, intradermal, or subcutaneous administration can include a sterile diluent, such as water, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose.

Pharmaceutical compositions for injection include sterile aqueous solutions (where water-soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). Antibacterial and antifungal agents include, for example, parabens, chlorobutanol, phenol, ascorbic acid and thimerosal. Isotonic agents, for example, sugars, polyalcohols such as manitol, sorbitol, sodium chloride may be included in the composition. Including an agent which delays absorption, for example, aluminum monostearate and gelatin can prolong absorption of injectable compositions.

The pharmaceutical formulations can be packaged in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the pharmaceutical carrier or excipient.

The compositions can be administered by any route compatible with a desired outcome. Thus, routes of administration include oral (e.g., ingestion or inhalation), intraperitoneal, intradermal, subcutaneous, intravenous, intraarterial, intracavity, intracranial, and parenteral. The compositions can also be administered using implants and microencapsulated delivery systems.

Compositions, including pharmaceutical formulations can further include particles or a polymeric substance, such as polyesters, polyamine acids, hydrogel, polyvinyl pyrrolidone, ethylene-vinylacetate, methylcellulose, carboxymethylcellulose, protamine sulfate, or lactide/glycolide copolymers, polylactide/glycolide copolymers, or ethylenevinylacetate copolymers. Cyclopropanecarboxylic acid, cyclopropanecarboxylic acid and derivatives and modified forms thereof can be entrapped in microcapsules, for example, by the use of hydroxymethylcellulose or gelatin-microcapsules, or poly (methylmethacrolate) microcapsules, respectively, or in a colloid drug delivery system.

In instances where cell, tissue or organ targeting is desired, a composition of the invention can of course be delivered to the target cell, organ or tissue by injection or infusion or the like. Targeting can be achieved by injection or infusion in practicing the methods of the invention. Targeting can also be achieved by using proteins that bind to a cell surface protein (e.g., receptor or matrix protein) present on the cell or population of cell types. For example, antibodies or antibody fragments (e.g., Fab region) that bind to a cell surface protein can be included in the delivery systems in order to facilitate cell, tissue or organ targeting. Viral coat proteins that bind particular cell surface proteins can be used for targeting. For example, naturally occurring or synthetic (e.g. recombinant) retroviral envelope proteins with known cell surface protein binding specificity can be employed in the liposomes in order to intracytoplasmically deliver cyclopropanecarboxylic acid, cyclopropanecarboxylic acid and derivatives and modified forms thereof into target cells, tissue or organs. Thus, delivery vehicles, including colloidal dispersion systems, can be made to have a protein coat in order to facilitate targeting or intracytoplasmic delivery of cyclopropanecarboxylic acid, cyclopropanecarboxylic acid and derivatives and modified forms thereof.

Prophylactic and therapeutic treatments of various physiological conditions or disorders treatable by increasing or improving glucose utilization in cells, tissue or organs of a patient by administering to the patient in need of such treatment, effective amounts of pharmaceutical compositions comprising substituted or unsubstituted cyclopropanecarboxylic acid, cyclopropanecarboxylic acid and cyclobutanecarboxylic acid derivative compounds represented by the Formulas (I) and (II) are contemplated.

Disorders or conditions that can be treated include, for example, ischemic/reperfusion injury, post myocardial infarction, angina, heart failure, a cardiomyopathy, peripheral vascular disease, diabetes, and lactic acidosis, or symptoms or side effects associated with heart surgery (e.g., open heart surgery, bypass surgery, heart transplant).

The pharmaceutical compositions comprising effective amounts of substituted or unsubstituted cyclopropanecarboxylic acid, cyclopropanecarboxylic acid and cyclobutanecarboxylic acid derivative compounds represented by the Formulas (I) and (II) are administered in a single daily dose, or the total daily dosage may be administered in divided doses several times daily. Furthermore, the pharmaceutical compositions may be administered as a single dose or over a period of time.

Patients that can be treated include all know kind of mammals, including non human primates (apes, gibbons, chimpanzees, orangutans, macaques), companion animals (dogs and cats), farm animals (horses, cows, goats, sheep, pigs), experimental animals (mouse, rat, rabbit, guinea pig), and humans.

The dosage regiment utilizing the pharmaceutical compositions according to the present invention is selected based on various factors such as type of physiological condition to be treated, age, weight, sex of the patient, severity of the conditions to be treated, the route of administration, and particular compound contained in the pharmaceutical composition. A physician or veterinarian of ordinary skill can readily determine and prescribed the effective amount of the pharmaceutical composition to prevent or to treat the specific physiological condition.

The daily dosage may be varied over wide range and can be such that the amount of the active compound selected from substituted or unsubstituted cyclopropanecarboxylic acid, cyclopropanecarboxylic acid and cyclobutanecarboxylic acid derivative compounds represented by the Formulas (I) and/or Formula (II) is sufficient to increase glucose utilisation in a cell, tissue or organ of a warm blooded animal and to achieve the desired effect of alleviating or preventing fatty acid-induced ischemic damage.

Kits are disclosed which contain substituted or unsubstituted cyclopropanecarboxylic acid, cyclopropanecarboxylic acid and derivatives and modified forms thereof represented by the Formula (I) and Formula (II), including pharmaceutical formulations, packaged into a suitable set. A kit typically includes a label or packaging insert including instructions for use, in vitro, in vivo, or ex vivo, of the components therein.

The term "packaging material" refers to a physical structure housing the components of the kit, such as cyclopropanecarboxylic acid, cyclopropanecarboxylic acid or derivatives or modified forms thereof. The packaging material can maintain the components sterilely, and can be made of material commonly used for such purposes (e.g., paper, corrugated fiber, glass, plastic, foil, ampules, etc.). The label or packaging insert can include appropriate written instructions, for example, practicing a treatment according the invention.

Kits of the invention therefore can additionally include instructions for using the kit components in a treatment of the invention. Instructions can include instructions for practicing any of the treatments of the invention described herein. Thus, for example, a kit can include a cyclopropanecarboxylic acid, cyclopropanecarboxylic acid or a derivative or modified form thereof in a pharmaceutical formulation in a container, pack, or dispenser together with instructions for administration to a human subject. Instructions may additionally include indications of a satisfactory clinical endpoint or any adverse symptoms that may occur, or any additional information required by the Food and Drug Administration for use in humans.

A kit may include instructions for increasing or improving glucose utilization in vitro, ex vivo or in vivo. In other embodiments, a kit includes instructions for treating a disorder associated with deficient or inefficient glucose utilization. In one aspect, the instructions comprise instructions for treating a subject having or at risk of having ischemic/reperfusion injury, post myocardial infarction, angina, heart failure, a cardiomyopathy, peripheral vascular disease, diabetes, or lactic acidosis. In another aspect, the instructions comprise instructions for treating a subject having or at risk of having heart surgery (e.g., open heart surgery, bypass surgery, heart transplant and angioplasty).

The instructions may be on "printed matter," e.g., on paper or cardboard within the kit, or on a label affixed to the kit or packaging material, or attached to a vial or tube containing a component of the kit. Instructions may additionally be included on a computer readable medium, such as a disk (floppy diskette or hard disk), optical CD such as CD- or DVD-ROM/RAM, magnetic tape, electrical storage media such as RAM and ROM and hybrids of these such as magnetic/optical storage media.

Kits can additionally include a buffering agent, a preservative, or a stabilizing agent. Each component of the kit can be enclosed within an individual container and all of the various containers can be within a single package.

The present invention is further illustrated in the following examples wherein all parts, percentages, and ratios are in equivalents, all temperatures are in °C, and all pressures are atmospheric unless otherwise indicated.

### EXAMPLES

### EXAMPLE 1

### Preparation of Cyclopropanecarboxylic acid, 2- [2- (2-methoxy-ethoxy)-ethoxy]-ethyl ester

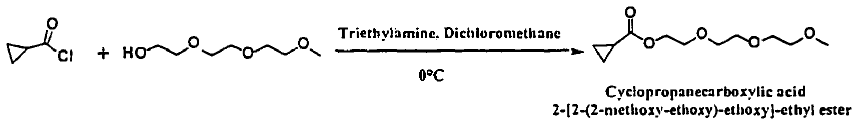

Triethylene glycol monomethyl ether (1.1 eq, 5.26 mmol, 0.84 ml) and triethylamine (1.1 eq, 5.26 mmol, 0.73 ml) were taken in a 10 ml round bottom flask and dichloromethane (3 ml) was added. This mixture was cooled to 0°C and then cyclopropanecarbonyl chloride (4.78 mmol, 0.5 g, 0.43 ml) was added in a dropwise fashion maintaining the temperature at 0°C with constant stirring.

A yellowish-orange solid was observed after some time. Stirring was continued for 1 hour at 0°C. The reaction was monitored by thin layer chromatography, and then quenched with saturated ammonium chloride solution. The mixture was then transferred to a separatory funnel, washed with 5% sodium bicarbonate (2 x 5 ml), 1:1 hydrochloric acid (2 x 5 ml) and then with brine (5 ml). The dichloromethane layer was separated from the aqueous layer, dried over anhydrous sodium sulphate, filtered, and evaporated *in vacuo* to give the title product as a pale yellow liquid. Purification was by flash chromatography and vacuum distillation (b.p. = 144°C, 3.0 mm of Hg) which afforded the pure product as a colorless liquid (527.0 mg, 48%).

The compound obtained was characterised by ¹H and ¹³C NMR, IR, and mass spectroscopy:
¹H NMR (300 MHz, CDCl₃) δ 4.2 (m, 2H), 3.68 (m, 2H), 3.64 (m, 6H), 3.52 (m, 2H), 3.36 (s, 3H), 1.62 (m, 1H), 0.99 (m, 2H), 0.84 (m, 2H); IR (CHCI₃) 2876.07, 1726.62, 1199.53, 1179.49, 1107.97 cm^{-1 13}C NMR (75.5 MHz, CDCl₃) δ 174.67, 71 .80, 70.44, 69.06, 63.45, 58.84, 12.65, 8.35; MS (ES, MNa⁺) : calculated for C₁₁H₂₀O₅Na 255.11, found 255.1.

### EXAMPLE 2 (Example 2 is a Reference Example)

### Preparation of Cyclobutanoylglycine (Cyclobutanecarbonyl-amino)-acetic acid)

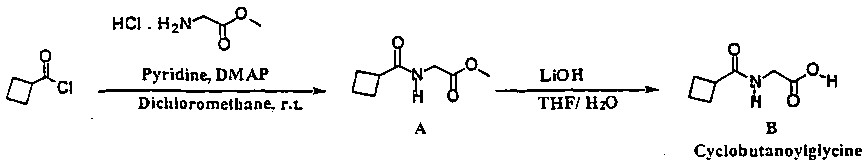

Methyl ester glycine hydrochloride (1 eq, 2.39 mmol, 300 mg) and pyridine (2 eq, 4.78 mmol, 0.39 ml) were suspended in (5 ml) of dichloromethane followed by addition of DMAP (1.5 eq, 218.5 mg) in one portion; the reaction mixture was stirred for 30 minutes at room temperature. After 30 minutes, cyclobutanecarbonyl chloride (2 eq, 4.77 mmol, 0.54 ml) was added slowly and the reaction mixture was stirred for 4 hours at room temperature. The solvent was evaporated in vacuo and the residue extracted with ethyl acetate. The ethyl acetate layer was dried and concentrated to dryness. The crude material obtained was purified by flash chromatography to yield pure compound **A** (358 mg, 87%).

To a solution of **A** in (6 ml) THF, was added lithium hydroxide (1.1 eq, 2.3 mmol, 2.3 ml, 1M) at room temperature and the reaction mixture was stirred for 1.5 hours. The reaction mixture was then concentrated in vacuo and acidified to pH = 3 with 2N HCl. The crude product was then extracted with ethyl acetate and purified by recrystallization, using an ethyl acetate/hexane mixture. The product obtained after recrystallization was further purified by flash chromatography and again recrystallization to give the title compound **B** as a white solid (196 mg, 59%).

The compound obtained was characterized by ¹H and ¹³C NMR, IR, and mass spectroscopy:
¹HNMR (300 MHz, CD₃OD) δ 3.87 (s, 2H), 3.14 (quintet, 1H), 1.84-2.2 (m, 6H); IR (USCOPE) 3313.01, 3098.14, 2986.18, 2940.41, 2525.15, 2469.13, 2435.25, 1738.49, 1620.96, 1566.87, 1486.61, 1346.65, 1264.23 cm⁻¹; ¹³C NMR (75.5 MHz, CD₃OD) δ 178.20, 173.12, 41.69, 40.61, 26.12, 19.01; HRMS (ES, MNa⁺): calculated for C₇H₁₁NO₃Na 180.06311, found 180.06290.

### EXAMPLE 3

### Preparation of Cyclobutanecarboxylic acid, 2-[2-(2-methoxcy-ethoxy)-ethoxy]-ethyl ester

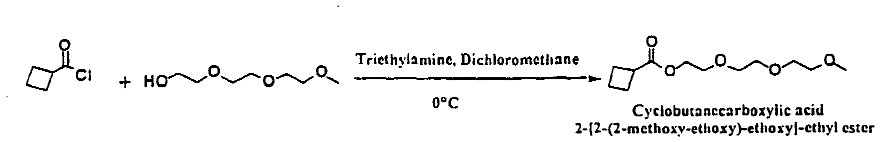

Triethylene glycol monomethyl ether (1.1 eq, 4.64 mmol, 0.74 ml) and triethylamine (1.1 eq, 4.64 mmol, 0.65 ml) were taken in a 25 ml round bottom flask and dichloromethane (3 ml) was added. This mixture was cooled to 0°C and then cyclobutanecarbonyl chloride (4.22 mmol, 0.5 g, 0.48 ml) was added in a dropwise fashion maintaining the temperature at 0°C with constant stirring (vigorous reaction).

A pink colored solution was observed after some time. An extra 4 ml of dichoromethane was added to maintain proper stirring (the reaction mixture became thick). Stirring was continued for 1 hour at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml) and then with brine (5 ml). The dichloromethane layer was separated from the aqueous layer, dried over anhydrous sodium sulphate, filtered, and evaporated in vacuo to give the title product as a pale yellowish-pink liquid. The liquid was purified by flash chromatography and vacuum distillation (b.p. = 189°C, 3.0 mm of Hg) to yield the pure product as a colorless liquid (679.6 mg, 65.34%).

The product was characterized by ¹H and ¹³C NMR, IR and mass spectroscopy:
¹HNMR (300 MHz, CDCl₃) δ 4.18 (m, 2H), 3.4 (m, 2H), 3.6 (m, 6H), 3.5 (m, 2H), 3.32 (s, 3H), 3.1 (quintet, 1H), 2.2 (m, 4H), 1.86 (m, 2H); IR (CDCI₃) 2946.38, 2870.68, 1730.73, 1179.35, 1109.59 cm⁻¹; ¹³C NMR (75.5 MHz, CDCI₃) δ 175.45, 71.93, 70.58, 69.21, 63.40, 59.01, 38.0, 25.24, 18.38; MS (ES, MNa⁺) : calculated for C₁₂H₂₂O₅Na 269.13, found 269.1.

### EXAMPLES 4, 6 to 14, 18 and 19 Example 19 is a Reference Example

### General Procedure for the preparation of certain cyclopropanecarboxylic acid and cyclobutanecarboxylic acid derivatives

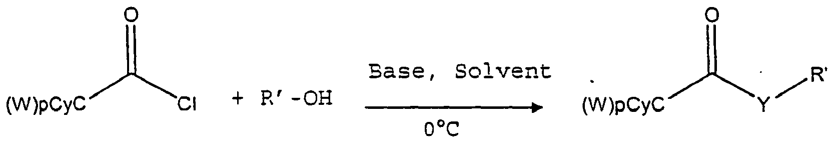

where W, Cyc, and p are as defined for Formula (I) and Y is O such that R'-YH is an alcohol, where R' is: 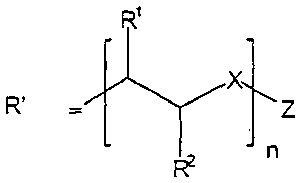 where R¹, R², Z and n are defined in connection with Formula (Ia); using triethylamine or pyridine as a base and dichloromethane as a solvent.

Following the procedures described in Example 1 and Example 3 and using the appropriate starting alcohol and cycloalkyl cyclopropane carboxylic acid chloride materials, (the appropriate starting alcohols were used in place of triethylene glycol monomethyl ether), the noted cyclopropanecarboxylic acid and of cyclobutanecarboxylic acid derivatives respectively were prepared (see Table I). The compounds prepared, cycloalkyl carbonylchloride and alcohol starting materials used for their preparation and their molecular weights are summarized in Table 1.

The compounds were characterized by ¹H NMR, ¹³C NMR, IR and mass spectroscopy.

### EXAMPLE 5 (Example 5 is a Reference Example)

### Preparation of Cyclopropanoylalanine

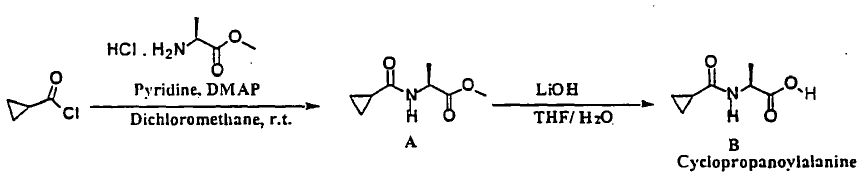

The procedure of Example 2 was followed except that 2.5 equivalents of pyridine was used instead of 2 equivalents, cyclopropanecarbonyl chloride was used in place of cyclobutanecarbonylchloride and methyl ester alanine hydrochloride was used in place of methyl ester glycine hydrochloride.

Purified compound **B** (321 mg, 87%) was characterized by ¹H and ¹³C NMR, IR, and mass spectroscopy.
¹HNMR (300 MHz, CD₃OD) δ 8.25 (br s, 1H), 4.38 (m, 1H), 3.25 (s, 1H), 1.64 (m, 1H), 1.39 (dd, 3H), 0.7- 0.9 (m, 4H); IR (USCOPE) 3323.78, 3020.25, 2645.76, 1791.56, 1729.13, 1632.33, 1537.27, 1406.24, 1281.02 cm⁻¹; ¹³C NMR (75.5 MHz, CD₃ OD) δ 176.28, 176.18, 49.38, 17.77, 14.59, 7.41, 7.33; HRMS (ES, M): calculated for C₇H₁₂NO₃ 158.08117, found 158.08123.

### EXAMPLE 15

### Preparation of Cyclopropanecarboxylic acid, 2-ethoxy-ethyl ester

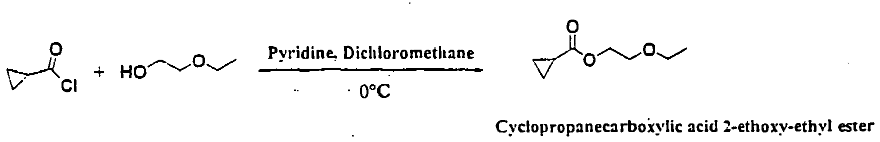

2-Ethoxy-ethanol (1.1 eq, 5.26 mmol, 0.47 g, 0.51 ml) and pyridine (1.1 eq, 5.26 mmol, 0.42 g, 0.43 ml) were taken in a 25 ml round bottom flask and dichloromethane (6 ml) was added. This mixture was cooled to 0°C and then cyclopropanecarbonyl chloride (4.78 mmol, 0.5 g, 0.43 ml) was added in a dropwise fashion maintaining the temperature at 0°C with constant stirring.

An orange-yellow colored solution was observed after sometime. Stirring was continued for 1 hour at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml), and then with brine (5 ml). The dichloromethane layer was separated from the aqueous layer, dried over anhydrous magnesium sulphate, filtered, and evaporated in vacuo to give the title product as a pale yellowish-orange liquid. Purification was by flash chromatography and vacuum distillation (b.p. = 43°C, 2.8 mm of Hg) which afforded the pure product as a colorless liquid (515.8mg, 55.4 %).

Characterization was done by NMR (¹H and ¹³C), IR, and mass spectroscopy:
¹HNMR (400 MHz, CDCl₃) δ 4.22 (m, 2H), 3.62 (m, 2H), 3.53 (q, 2H), 1.64 (m, 1H), 1.2 (t, 3H), 0.98 (m, 2H), 0.84 (m, 2H) ; MS (ES, M+Na): calculated for C₈H₁₄O₃Na 181.19, found 181.1 ; IR (CH₂Cl₂) 2976.37, 2869.55, 1728.78, 1455.55, 1177.86 cm⁻¹; ¹³CNMR (125 MHz, CDCl₃) δ 174.76, 68.39, 66.60, 63.73, 15.17, 12.91, 8.62.

### EXAMPLE 16

### Preparation of Cyclobutanecarboxylic acid 2-ethoxy-ethyl ester

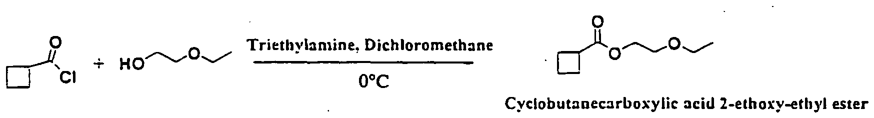

2-Ethoxy-ethanol (1.1eq, 4.64 mmol, 0.42 g, 0.45 ml) and triethylamine (1.1 eq, 4.64 mmol, 0.47 g, 0.65 ml) were taken in a 25 ml round bottom flask and dichloromethane (6 ml) was added. This mixture was cooled to 0°C and then cyclobutanecarbonyl chloride (4.22 mmol, 0.5 g, 0.48 ml) was added in a dropwise fashion maintaining the temperature at 0°C with constant stirring.

An orange-yellow colored solution was observed after sometime. Stirring was continued for 1 hour at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml), and then with brine (5 ml). The dichloromethane layer was separated from the aqueous layer, dried over anhydrous magnesium sulphate, filtered, and evaporated in vacuo to give the title product as a pale yellow liquid. Purification was attempted by flash chromatography and vacuum distillation (b.p. = 48°C, 2.8 mm of Hg) which afforded the pure product as a colorless liquid (421.3 mg, 57.7 %).

Characterization was done by NMR (¹H and ¹³C), IR, and mass spectroscopy:
¹HNMR (500 MHz, CDCl₃) δ 4.18 (m, 2H), 3.58 (m, 2H), 3.48 (q, 2H), 3.14 (m, 1H) 2.2 (m, 4H), 1.9 (m, 2H), 1.17 (t, 3H); MS (ES, M+Na): calculated for C₉H₁₆O₃Na 195.11, found 195.1; IR (CH₂Cl₂) 2976.99, 2949.17, 1732.12, 1444.39, 1175.39 cm⁻¹;
¹³CNMR (125 MHz, CDCl₃) δ 175.33, 68.38, 66.58, 63.51, 38.06, 25.32, 28.47, 15.16.

### EXAMPLE 17

### Preparation of cyclopropanecarboxylic acid, 2-isopropoxymethyl ester

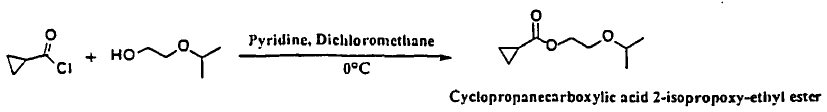

2-Isopropoxy-ethanol (1.1eq, 5.26 mmol 0.55 g, 0.61 ml) and pyridine (1.1 eq, 5.26 mmol, 0.42 g, 0.43 ml) were taken in a 25 ml round bottom flask and dichloromethane (6 ml) was added. This mixture was cooled to 0°C and then cyclopropanecarbonyl chloride (4.78 mmol, 0.5 g, 0.43 ml) was added in a dropwise fashion maintaining the temperature at 0°C with constant stirring.

An orange-yellow colored solution was observed after sometime. An extra 2 ml of dichoromethane was added to maintain proper stirring (reaction mixture becomes thick). Stirring was continued for 1 hour at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml), and then with brine (5 ml). The dichloromethane layer was separated from the aqueous layer, dried over anhydrous magnesium sulphate, filtered, and evaporated *in vacuo* to give the title product as a pale yellowish-orange liquid. Purification was by flash chromatography and vacuum distillation (b.p. = 33°C, 2.9 mm of Hg) which afforded the pure product as a colorless liquid (630.2 mg, 76.40%).

Characterization of the resulting compound was done by ¹H and ¹³C NMR, IR, and mass spectroscopy:
¹HNMR (400 MHz, CDCl₃) δ 4.2 (m, 2H), 3.6 (m, 3H), 1.65 (m, 1H), 1.15 (d, 6H), 1.0 (m, 2H), 0.85 (m, 2H); IR (CH₂CI₂) 3015.93, 2972.88, 1729.05, 1454.97, 1177.85 cm⁻¹; ¹³C NMR (125 MHz, CDCI₃) δ 174.72, 71.93, 65.95, 64.0, 22.06, 12.92, 8.54; MS (ES, MNa⁺): calculated for C₉H₁₆O₃Na 195.09, found 195.0

### EXAMPLE 20 (Example 20 is a Reference Example)

### Preparation of Cyclobutanecarboxylic acid, 2-(2-cyclobutanecarbonyloxy-ethoxy)-ethyl ester

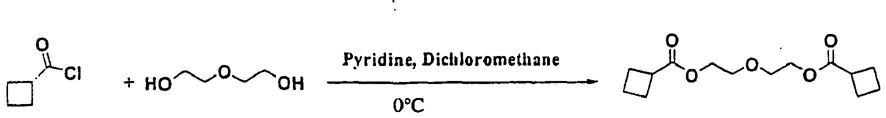

Diethylene glycol (0.5 eq, 1 mmol, 0.11 g) and pyridine (2.2 eq, 2.2 mmol, 0.174 g, 0.18 ml) were taken in a 10 ml round bottom flask and dichloromethane (4 ml) was added. This mixture was cooled to 0°C and then cyclobutanecarbonyl chloride (2.0 mmol, 0.24 g, 0.23 ml) was added in a dropwise fashion maintaining the temperature at 0°C with constant stirring.

A white thick suspension was observed after sometime. Stirring was continued for 1 hour at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml), and then with brine (5 ml). The dichloromethane layer was separated from the aqueous layer, dried over anhydrous magnesium sulphate, filtered, and evaporated *in vacuo* to give the title product as a pale yellowish liquid. Purification was by flash chromatography and vacuum distillation (b.p. = 113°C, 2.8 mm of Hg) which afforded the pure product as a colorless liquid (130.0 mg, 46.42 %).

Characterization was done by NMR (¹H and ¹³C), IR, and mass spectroscopy:
¹HNMR (500 MHz, CDCl₃) δ 4.2 (m, 4H), 3.63 (m, 4H), 3.14(m, 2H), 1.9 - 2.2(m, 12H); MS (ES, M+Na): calculated for C₁₄H₂₂O₅Na 293.15, found 293.0; IR (CH₂Cl₂) 2949.36, 2869.67, 1731.42, 1445.39, 1174.72 cm⁻¹; ¹³CNMR (125 MHz, CDCl₃) δ 175.26, 69.16, 63.32, 63.27, 38.05, 25.33, 18.49.

### EXAMPLE 21 (Example 21 is a Reference Example)

### Preparation of Cyclopropanecarboxylic acid, 2-[2-(2-cyclopropanecarbonyloxy-ethoxy)-ethoxy]-ethyl ester

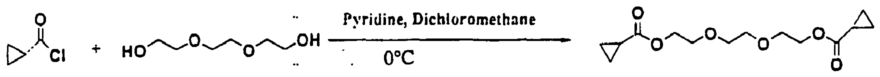

Triethylene glycol (1.6 mmol, 0.24 g) and pyridine (2.2 eq, 3.52 mmol, 0.28 g, 0.28 ml) were taken in a 10 ml round bottom flask and dichloromethane (5 ml) was added. This mixture was cooled to 0°C and then cyclopropanecarbonyl chloride (3.4 mmol, 0.36 g, 0.31 ml) was added in a dropwise fashion maintaining the temperature at 0°C with constant stirring.

A white, thick suspension was observed after sometime. Stirring was continued for 1 hour at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml), and then with brine (5 ml). The dichloromethane layer was separated from the aqueous layer, dried over anhydrous magnesium sulphate, filtered, and evaporated *in vacuo* to give the title product as a pale yellowish liquid. Purification was by flash chromatography and vacuum distillation (b.p. = 127°C, 2.8 mm of Hg) which afforded the pure product as a colorless liquid (234.5 mg, 50.97 %).

Characterization was done by NMR (¹H and ¹³C), IR, and mass spectroscopy:
¹HNMR (500 MHz, CDCl₃) δ 4.2 (m, 4H), 3.68 (m, 4H), 3.64(br s, 4H), 1. 62 (m, 2H), 0.97 (m,4H), 0.84 (m, 4H); MS (ES, M+Na) : calculated for C₁₄H₂₂O₆Na 309.14, found 309.0; IR (CH₂Cl₂) 3015.01, 2951.60, 2873.12, 1726.69, 1454.28, 1177.84 cm⁻¹; ¹³CNMR (125 MHz, CDCl₃) δ 175.0, 70.0, 69.0, 63.0, 13.0, 8.0.

### EXAMPLE 22 (Example 22 is a Reference Example)

### Preparation of Cyclobutanecarboxylic acid, 2-[2-(2-cyclobutanecarbonyloxy-ethoxy)- ethoxy]-ethyl ester

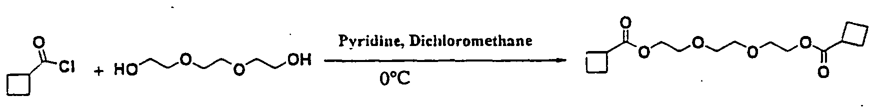

The procedure described in Example 21 was followed using the following amounts of these reagents: triethylene glycol (1.6 mmol, 0.24 g), pyridine (2.2 eq, 3.52 mmol, 0.28 g, 0.28 ml); and cyclobutanecarbonyl chloride (3.4 mmol, 0.40 g, 0.39 ml).

The compound was characterized by NMR (¹H and ¹³C), IR and mass spectroscopy:
¹HNMR (500 MHz, CDCl₃) δ 4.2 (m, 4H), 3.68 (m, 4H), 3.62(br s, 4H), 3.14(m, 2H), 2.1-2.3 (m, 8H), 1.8-2.0 (m, 4H); MS (ES, M+Na) : calculated for C₁₆H₂₆O₆Na 337.14, found 337.0; IR (CH₂Cl₂) 2948.71, 2869.27, 1731.17, 1445.80, 1175.60 cm⁻¹;
¹³CNMR (125 MHz, CDCl₃) δ 175.29, 70.59, 69.27, 63.39, 38.06, 25.33, 18.49.

### EXAMPLE 23 (Example 23 is a Reference Example)

### Preparation of Cyclopropanecarboxylic acid, 2-[{2-[bis(2-cyclopropanecarbonyloxy-ethyl)-amino]-ethyl}-(2-cyclopropanecarbonyloxy-ethyl)-amino]ethyl ester

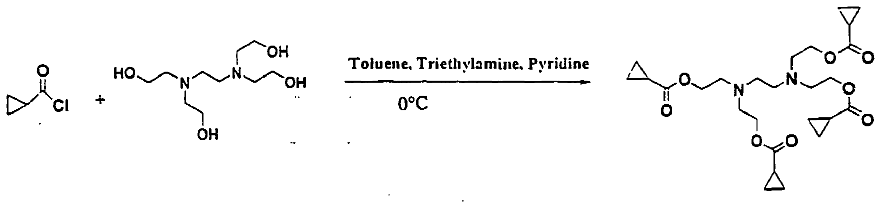

A solution of the diamine tetra-ol (1 eq, 4.23 mmol, 1.0 g), pyridine (1.0 eq, 4.23 mmol, 0.33 g, 0.34 ml) and triethylamine (5.0 eq, 0.021 mol, 2.12 g, 2.90 ml) was taken in a 25 ml round bottom flask and toluene (10 ml) was added. This mixture was cooled to 0°C and then cyclopropanecarbonyl chloride (0.019 mol, 1.98 g, 1.72 ml) was added in one shot with vigorous stirring, maintaining the temperature at 0°C.

A yellowish-white thick suspension was observed after sometime. Stirring was continued for 15 minutes at 0°C. The reaction was monitored by thin layer chromatography and then quenched with saturated ammonium chloride solution. The mixture was then transferred to a separatory funnel, and extracted with ethyl acetate (2 x 25 ml). The ethyl acetate layer was washed with brine (1 x 20 ml), dried over anhydrous magnesium sulphate, filtered, and evaporated *in vacuo* to give the title product as a pale yellowish liquid. Purification was by flash chromatography which afforded the pure product as a colorless liquid (900.0 mg, 42.0 %).

Characterization was done by NMR (¹H and ¹³C), IR, and mass spectroscopy:
¹HNMR (300 MHz, CDCl₃) δ 4.13 (t, 8H), 3.3 (m, 3H), 2.82 (t, 8H), 2.68 (s, 4H) 1.64 (m,4H), 0.88 (m, 16H); MS (ES, M+H): calculated for C₂₆H₄₀N₂O₈ +H 509.29, found 509.0; IR (CH₂Cl₂) 3014.69, 2958.19, 2826.89, 1725.90, 1452.31, 1173.00 cm⁻¹; ¹³CNMR (125 MHz, CDCl₃) δ 174.59, 62.62, 53.55, 53.29, 12.92, 8.50.

### Example 24 (Example 24 is a Reference Example)

### Preparation of Cyclopropanecarboxylic acid (2-isopropoxy-ethyl)-amide

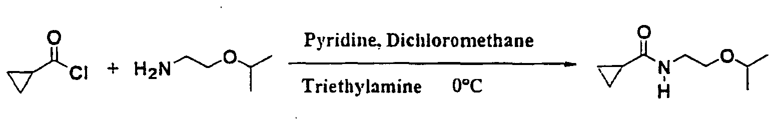

A solution of the amino ether (1.1 eq, 5.26 mmol, 0.54 g, 0.64 ml), pyridine (0.5 eq, 2.39 mmol, 0.19 g, 0.19 ml), triethylamine (1.1 eq, 5.26 mmol, 0.53 g, 0.73 ml) was taken in a 10 ml round bottom flask and dichloromethane (6 ml) was added. This mixture was cooled to 0°C and then cyclopropanecarbonyl chloride (4.78 mmol, 0.5 g, 0.43 ml) was added in a dropwise fashion, maintaining the temperature at 0°C.

A white precipitate was observed after sometime. Stirring was continued for 30 minutes at 0°C. The reaction was monitored by thin layer chromatography and then quenched with saturated ammonium chloride solution. The mixture was then transferred to a separatory funnel, washed with water (1 x 10 ml), brine (2 x 10 ml), dried over anhydrous magnesium sulphate, filtered, and evaporated in vacuo to give the title product as a colorless liquid. Purification was by flash chromatography and vacuum distillation (b.p. =106°C, 1.4 mm of Hg) which afforded the pure product as a colorless liquid (493.8 mg, 60.22 %).

Characterization was done by NMR (¹H and ¹³C), IR, and mass spectroscopy:
¹HNMR (300 MHz, CDCl₃) δ 6.0 (br s, 1H), 3.57 (m, 1H), 3.44 (m, 4H), 1.32 (m, 1H) 1.14 (d, 6H), 0.94 (m, 2H), 0.7 (m, 2H); MS (EI, M⁺): calculated for C₉H₁₇NO₂ 171.12, found 171.12; IR (CH₂Cl₂) 3299.39, 3092.61, 2971.98, 2868.14, 1644.61, 1552.31, 1197.34 cm⁻¹; ¹³CNMR (125 MHz, CDCl₃) δ 173.41, 71.76, 66.68, 39.83, 22.10, 14.72, 7.07.

### EXAMPLE 25 (Example 25 is a Reference Example)

### Preparation of (±)-trans-2-Phenyl-cyclopropanecarboxylic acid 2-isopropoxy-ethyl ester

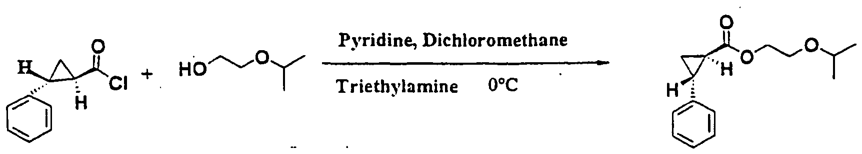

2-Isopropohy-ethanol (1.1 eq, 3.04 mmol, 0.32 g, 0.35 ml), pyridine (0.5 eq, 1.38 mmol, 0.11 g, 0.11 ml), triethylamine (1.1 eq, 3.04 mmol, 0.31 g, 0.43 ml) were taken in a 10 ml round bottom flask and dichloromethane (6 ml) was added. This mixture was cooled to 0°C and then (±)-trans-2-phenyl-cyclopropanecarbonyl chloride (2.76 mmol, 0.5 g, 0.43 ml) was added in a dropwise fashion, maintaining the temperature at 0°C.

A white precipitate was observed after sometime. Stirring was continued for 30 minutes at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml), and then with brine (5 ml). The dichloromethane layer was separated from the aqueous layer, dried over anhydrous magnesium sulphate, filtered, and evaporated *in vacuo* to give the title product as a pale yellowish liquid. Purification was by flash.chromatography which afforded the pure product as a colorless liquid (450.0 mg, 65.0 %). The above compound is racemic as determined by chiral HPLC (chiralcel OJ column) 2% Isopropanol in hexane. UV λₘₐₓ = 278 nm (flow = 1 ml/ min).

Characterization was done by NMR (¹H and ¹³C), IR, and mass spectroscopy:
¹HNMR (300 MHz, CDCl₃) δ 7.07- 7.27 (m, 5H), 4.22 (m, 2H), 3.61 (m, 3H), 2.51 (m, 1H) 1.93 (m, 1H), 1.58 (m, 1H), 1.26 (m, 1H), 1.53 (d, 6H); MS (EI, M⁺): calculated for C₁₅H₂₀O₃ 248.14, found 248.14; IR (CH₂Cl₂) 3063.09, 3030.28, 2971.95, 2867.20, 1727.02, 1151.71 cm⁻¹; ¹³CNMR (125 MHz, CDCl₃) δ 173.27, 139.92, 128.36,126.39, 126.10, 72.01, 65.94, 64.33, 26.44, 24.17, 22.10, 17.26.

### EXAMPLE 26 (Example 26 is a Reference Example)

### Preparation of (±)-trans-2-Phenyl-cyclopropanecarboxylic acid 2

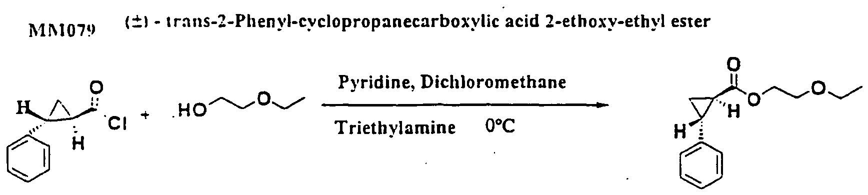

This compound was prepared using the procedure described in Example 25 and using the following reagents: 2-ethoxy-ethanol (1.1 eq, 3.05 mmol, 0.27 g, 0.30 ml) pyridine (0.5 eq, 1.39 mmol, 0.11 g, 0.11 ml), triethylamine (1.1 eq, 3.05 mmol, 0.31 g, 0.43 ml); and (±)-trans-2-phenyl- cyclopropanecarbonyl chloride (2.77 mmol, 0.5 g, 0.43 ml).

The compound was characterized by NMR (¹H and ¹³C), IR and mass spectroscopy:
¹HNMR (300 MHz, CDCl₃) δ 7.06- 7.28 (m, 5H), 4.24 (m, 2H), 3.62 (m, 2H), 3.52 (q, 2H) 2.51 (m, 1H), 1.94 (m, 1H), 1.58 (m, 1H), 1.29 (m, 1H), 1.19 (t, 3H); MS (EI, M⁺): calculated for C₁₄H₁₈O₃ 234.12, found 234.12; IR (CH₂Cl₂) 2975.07, 2868.63, 1726.37, 1175.66 cm⁻¹; ¹³CNMR (125 MHz, CDCl₃) δ 173.26, 139.90, 128.37,126.40, 126.08, 68.36, 66.65, 64.04, 26.48, 24.13, 17.35, 15.2

### EXAMPLE 27 (Example 27 is a Reference Example)

### Preparation of 1-Phenyl-cyclopropanecarboxylic acid 2-ethoxy-ethyl ester

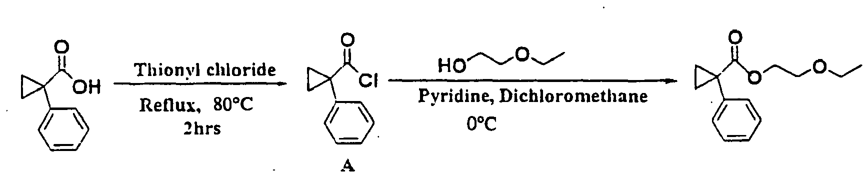

1-Phenyl-cyclopropanecarboxylic acid (0.5 g, 3.1 mmol), was dissolved in thionyl chloride (10 eq, 0.031 mol, 3.68 g, 2.3 ml), and refluxed at 80°C for 1.5 hours. Then excess of thionyl chloride was evaporated on the rotary evaporator which yielded a dark- yellowish liquid (1-phenylcyclopropanecarbonyl chloride A), which was then cooled to 0°C, under argon.

2-Ethoxy-ethanol (1.1 eq, 3.41 mmol, 0.31 g, 0.33 ml) and pyridine (1.2 eq, 3.41 mmol, 0.27g, 0.28 ml) was taken in a 25 ml round bottom flask and dichloromethane (10 ml) was added. This mixture was cooled to 0°C and then A was added in a dropwise fashion maintaining the temperature at 0°C. Stirring was continued for 30 minutes at 0°C. The reaction was monitored by thin layer chromatography and then quenched with saturated ammonium chloride solution. The mixture was then transferred to a separatory funnel, washed with 5% sodium bicarbonate (2 x 5 ml), 1:1 hydrochloric acid (2 x 5 ml), and then with brine (5 ml). The dichloromethane layer was separated from the aqueous layer, dried over anhydrous magnesium sulphate, filtered, and evaporated in vacuo to give the title product as a pale yellowish liquid. Purification was by flash chromatography and vacuum distillation (b.p. = 93°C, 2.4 mm of Hg), which afforded the pure product as a colorless liquid (437.7 mg, 60.62%)

Characterization was done by NMR (¹H and ¹³C), IR, and mass spectroscopy:
¹HNMR (300 MHz, CDCl₃) δ 7.2- 7.35 (m, 5H), 4.14 (m, 2H), 3.51 (m, 2H), 3.35 (q, 2H) 1.60 (dd, 2H), 1.18 (dd, 2H), 1.1 (t, 3H); MS (EI, M⁺): calculated for C₁₄H₁₈O₃ 234.12, found 234.12; IR (CH₂Cl₂) 2958.04, 2837.41, 1676.09, 1600.65, 1288.07 cm⁻¹; ¹³CNMR (125 MHz, CDCl₃) δ 174.28, 139.40, 130.41, 127.97, 127.00, 68.11, 66.57, 64.43, 29.19, 16.59, 15.23.

### Note:

For the compounds of Examples 1 to 27, the solvent system used for flash chromatography was ethyl acetate/hexane, unless otherwise specified.

**TABLE 1**

| Example | Compound | Molecular Weight | Starting Carbonyl Chloride | Starting R' - YH compound |
|---|---|---|---|---|
| 1 MM054 | 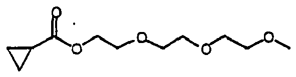 | 232.28 | P* | 2-[2-(2-Methoxy-ethoxy)-ethoxy] -ethanol |
| | Cyclopropanecarboxylic acid 2-[2-(2-methoxy-ethoxy)-ethoxy]-ethyl ester | | | |
| 2 MM055 | 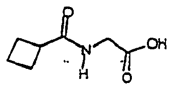 | 157.17 | B** | Methyl ester glycine hydrochloride (Amino Acid) |
| | (Cyclobutancarbonyl-amino)-acetic acid | | | |
| 3 MM056 | 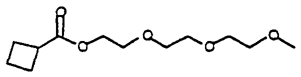 | 246.31 | B | 2-[2-(2-Methoxy-ethoxy)-ethoxy]-ethanol |
| | Cyclocarboxylic acid 2-[2-(2-methoxy-ethoxy)-ethoxy)-ethoxy]-ethyl ester | | | |
| 4 MM057 | 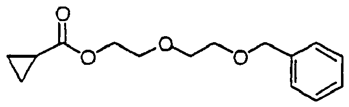 | 264.31 | P | 2-(2-Benzyloxy-ethoxy)-ethanol |
| | 1-cyclopropanecarboxylic acid 2-(2-benzyloxy-ethoxy)-ethyl ester | | | |
| 5 MM058 | 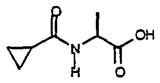 | 157.17 | P | Methyl ester alanine hydrochloride (Amino Acid) |
| | 2-(Cyclopropanecarbonyl-amino)-propionic acid | | | |
| 6 MM059 | 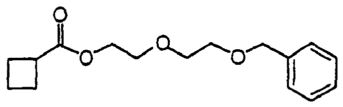 | 278.34 | B | 2-(2-Benzyloxy-ethoxy)-ethanol |
| | Cyclobutanecarboxylic acid 2-(2-benzyloxy-ethyl)ester ester | | | |
| 7 MM060 | 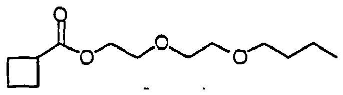 | 244.32 | B | 2-(2-Butoxy-ethoxy)-ethanol |
| | Cyclobutanecarboxylic acid, 2-(2-butoxy-ethoxy)-ethyl ester | | | |
| 8 MM061 | 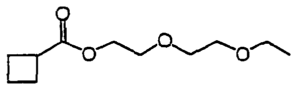 | 216.27 | B | 2-(2-ethoxy-ethoxy)-ethanol |
| | Cyclobutanecarboxylic acid. 2-(2-ethoxy-ethoxy)-ethyl ester | | | |
| 9 MM062 | 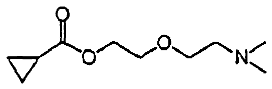 | 201.36 | P | 2-(2-dimethylamino -ethoxy)-ethanol |
| | Cyclopropanecarboxylic acid 2-(2-dimethylamino-ethoxy)-ethyl ester | | | |
| 10 MM063 | 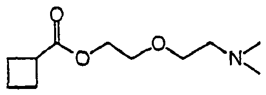 | 215.29 | B | 2- (2-dimethylamino -ethoxy)-ethanol |
| | Cyclobutanecarboxlic acid 2-(2-dimethylamino-ethoxy)-ethyl ester | | | |
| 11 Mum064 | 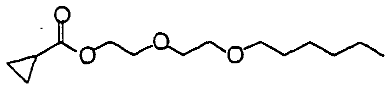 | 258.35 | P | 2-(2-hexyloxy-ethoxy)-ethanol |
| | Cyclopropanecarboxylic acid 3-(2-hexyloxy-ethoxy)-ethyl ester | | | |
| 12 MM065 | 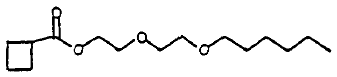 | 272.39 | B | 2-(2-hexyloxy-ethoxy)-ethanol |
| | Cyclobutanecarboxylic acid 2-(2-hexyloxy-ethoxy)-ethyl ester | | | |
| 13 MM066 | 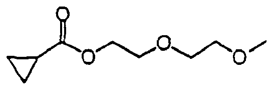 | 188.23 | P | 2-(2-methoxy-ethoxy)-ethanol |
| | Cyclopropanecarboxylic acid 2-(2-methoxy-ethoxy)-ethyl ester | | | |
| 14 MM067 | 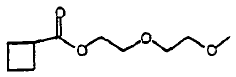 | 202.25 | B | 2-(2-methoxy-ethoxy)-ethanol |
| | Cyclobutanecarboxylic acid 2-(2-methoxy-ethoxy)-ethyl ester | | | |
| 15 MM068 | 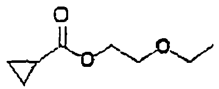 | 158.20 | P | 2-ethoxy-ethanol |
| | Cyclopropanecarboxylic acid 2-ethoay-ethyl ester | | | |
| 16 MM069 | 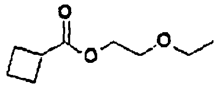 | 172.23 | B | 2-ethoxy-ethanol |
| | Cyclobutanecarboxylic acid 2-ethoxy-ethyl ester | | | |
| 17 MM070 | 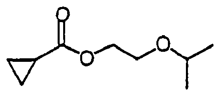 | 172.23 | P | 2-Isopropoxy-ethanol |
| | Cyclopropanecarboxylic acid 2-isopropoxy-ethyl ester | | | |
| 18 MM071 | 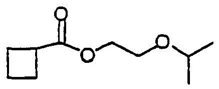 | 186.25 | B | 2-Isopropoxy-ethanol |
| | Cyclobutanecarboxylic acid 2-isopropoxy-ethyl ester | | | |
| 19 MM072 | 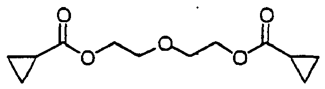 | 242.27 | P | 2-(2-hydroxy-methoxy)-ethanol |
| | Cyclopropanecarboxylic acid, 2-2(cycloplopronecarbonyloxy-ethoxy)-ethyl ester | | | |
| 20 MM073 | 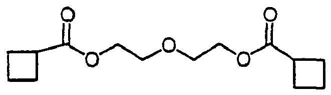 | 270.32 | B | 2-(2-Hydroxy-ethoxy)-ethanol |
| | Cyclobutanecarboxylic acid, 2-(2-cyclobutanecarbonyloxy-ethoxy)ethyl ester | | | |
| 21 MM014 | 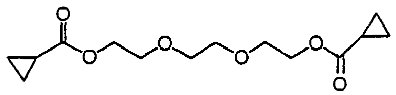 | 286-32 | P | 2-[2-(2-Hydroxy-ethoxy)-ethoxyl-methanol |
| | Cyclopropanecarboxylic acid, 2-[2-(2-cyclopropanecarbonyloxy-ethoxy)-ethoxy}ethyl ester | | | |
| 22 MM075 | 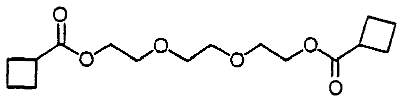 | 314.37 | B | 2-[2-(2-Hydroxy-ethoxy)-ethoxy]-ethanol |
| | Cyclobutanecarboxylic acid,2-(2-(2-cyclobutanecarbonyloxy-ethoxy)-ethoxy)ethyl ester | | | |
| 23 MM076 | 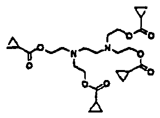 | 508.60 | P | N, N, N' , N' -Tetrakis(2-hydroxyethyl) ethyl-enediamine |
| | Cyclopropanecarboxylic acid 2-[{2-[bis(2-cyclopropanecarbonyloxy-ethyl)-amino]-ethyl}-(2-cyclopropanecarbonyloxy-ethyl)-amino] ethyl ester | | | |
| 24 MM077 | 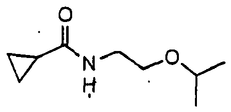 | 171.24 | P | 2-Aminoethyl isopropyl ether |
| | -Cyclopropanecarboxylic acid (2-isopropoxy-ethyl)-amide | | | |
| 25 MM078 | 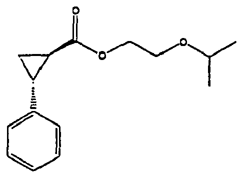 | 248.32 | trans-2-phenyl-cyclopro-panecar-bonyl chloride | 2-Isopropoxy-ethanol |
| | trans-2-Phenyl-cyclopropanecarboxylic acid 2-isopropoxy-ethyl ester | | | |
| 26 MM079 | 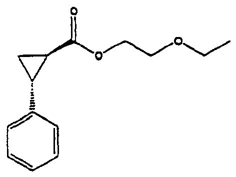 | 234.29 | trans-2-phenyl-cyclopro-panecar-bonyl chloride | 2-ethoxy-methanol |
| | trans-2-Phenyl-cyclopropanecarboxylic acid 2-ethoxy-ethyl ester | | | |
| 27 MM080 | 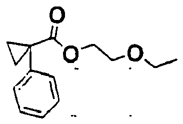 | 234.29 | 1-phenyl-cyclo-propane-carbonyl chloride | 2-methoxy-ethanol |
| | 1-Phenyl-cyclopopanecarboxylic acid 2-ethoxy-ethyl ester | | | |

| | | | | |
|---|---|---|---|---|
| a. P* Cyclopropanecarbonyl chloride b. B** Cyclobutanecarbonyl chloride | | | | |

### EXAMPLE A

Glucose oxidation stimulation in untreated myocardium cells and myocardium cells treated with cyclopropanecarboxylic acid, 2-[2-(2- methoxy-ethoxy)-ethoxy]-ethyl ester.
Rat hearts were cannulated for isolated working heart 60 min aerobic perfusions as described in J Pharmacol Exp Ther. 1993; 264:135 -144, the entire disclosure of which is incorporated herein by reference.

Male Sprague-Dawley rats (0.3-0.35 kg) were anesthetized with pentobarbital sodium (60 mg/kg IP) and hearts were quickly excised, the aorta was cannulated and a retrograde perfusion at 37°C was initiated at a hydrostatic pressure of 60 mm Hg. Hearts were trimmed of excess tissue, and the pulmonary artery and the opening to the left atrium were then cannulated. After 15 min of Langendorff perfusion, hearts were switched to the working mode by clamping the aortic inflow line from the Langendorff reservoir and opening the left atrial inflow line. The perfusate was delivered from an oxygenator into the left atrium at a constant preload pressure of 11 mm Hg. Perfusate was ejected from spontaneously beating hearts into a compliance chamber (containing 1 ml of air) and into the aortic outflow line. The afterload was set at a hydrostatic pressure of 80 mm Hg. All working hearts were perfused with Krebs'-Henseleit solution containing calcium 2.5 mmol/L, glucose 5.5 mmol/L, 3% bovine serum albumin (fatty acid free, initial fractionation by heat shock, Sigma), and with 1.2 mmol/L palmitate. Palmitate was bound to the albumin as described in J Bio Chem. 1992; 267:3825-3831, the entire disclosure of which is incorporated herein by reference.

The perfusate was recirculated, and pH was adjusted to 7.4 by bubbling with a mixture containing 95% O₂ and 5% CO₂.

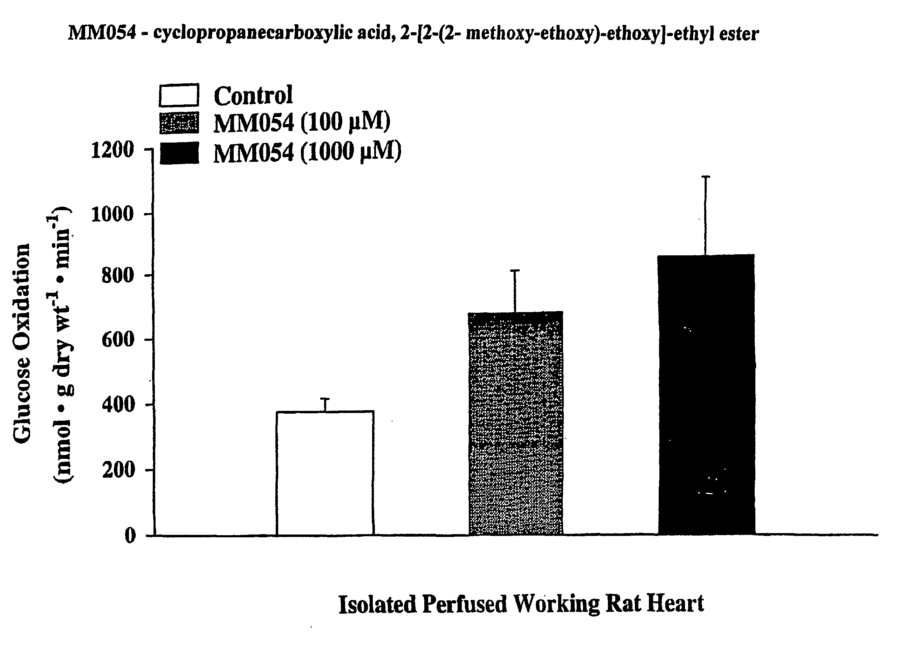

Spontaneously beating hearts were used in all perfusions. Heart rate and aortic pressure were measured with a Biopac Systems Inc. blood pressure transducer connected to the aortic outflow line. Cardiac output and aortic flow were measured with Transonic T206 ultrasonic flow probes in the preload and afterload lines, respectively. Coronary flow was calculated as the difference between cardiac output and aortic flow. Cardiac work was calculated as the product of systolic pressure and cardiac output.
*Measurement of Glucose Oxidation* : Glucose oxidation was measured simultaneously by perf using hearts with [U ⁻¹⁴C] glucose according to the procedures discussed in Saddik M, et al., J Bio Chem. 1992; 267:3825 -3831. The entire disclosure of this reference is incorporated herein by reference. The total myocardial ¹⁴CO₂ production was determined at 10 -min intervals from the 60 -min aerobic period. Glucose oxidation rates were determined by quantitative measurement of ¹⁴CO₂ production as described in Barbour RL, et al., Biochemistry 1984; 1923:6503 -6062. The entire disclosure of this reference is incorporated herein by reference. ¹⁴CO₂ production for the control group were compared with the ¹⁴CO₂ production for the group treated with cyclopropanecarboxylic acid, 2 -[2-(2- methoxy-ethoxy)-ethoxy]-ethyl ester. Results are shown on Figure 1 and TABLE 2.

### Example B Examples which use compounds of Reference Examples in point (3) are not included in the claims. The Example of point (4) is not included in the claims.

### (1) Glucose oxidation stimulation in myocardium cells treated with cyclobutanecarboxylic acid, 2-[2-(2- methoxyethoxy)-ethoxy]-ethyl ester.

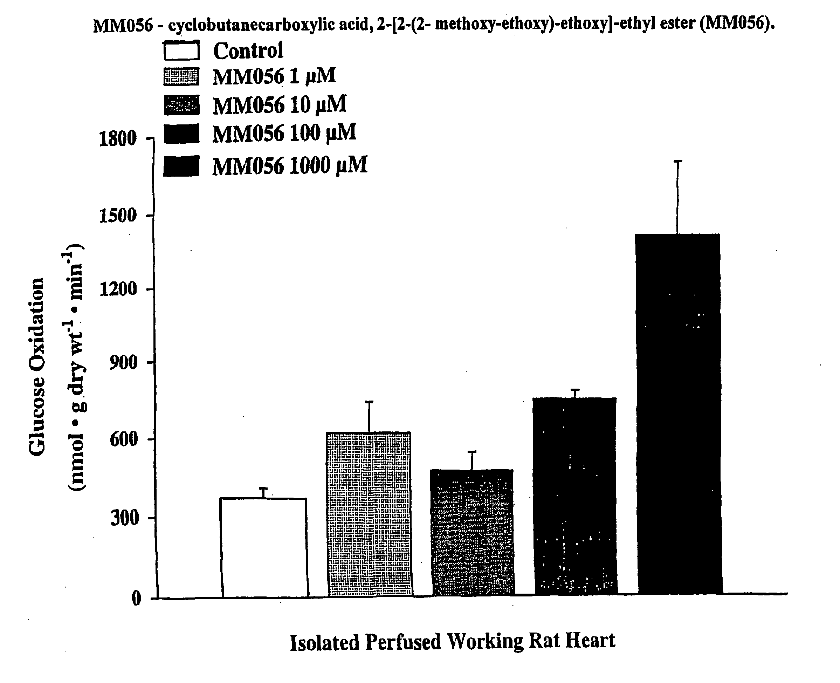

The procedure of Example A for was followed except that cyclobutanecarboxylic acid, 2-[2-(2- methoxy-ethoxy)-ethoxy]-ethyl ester in 1 µM, 10 µM, 100 µM and 1000 µM amounts was added to the buffer in place of the cyclopropanecarboxylic acid, 2-[2-(2- methoxy-ethoxy)-ethoxy]-ethyl ester. The results are illustrated in Figure 2 and TABLE 2.

### (2) Glucose oxidation stimulation in myocardium cells treated with cyclopropanecarboxylic acid, 2-isopropoxy ethyl ester.

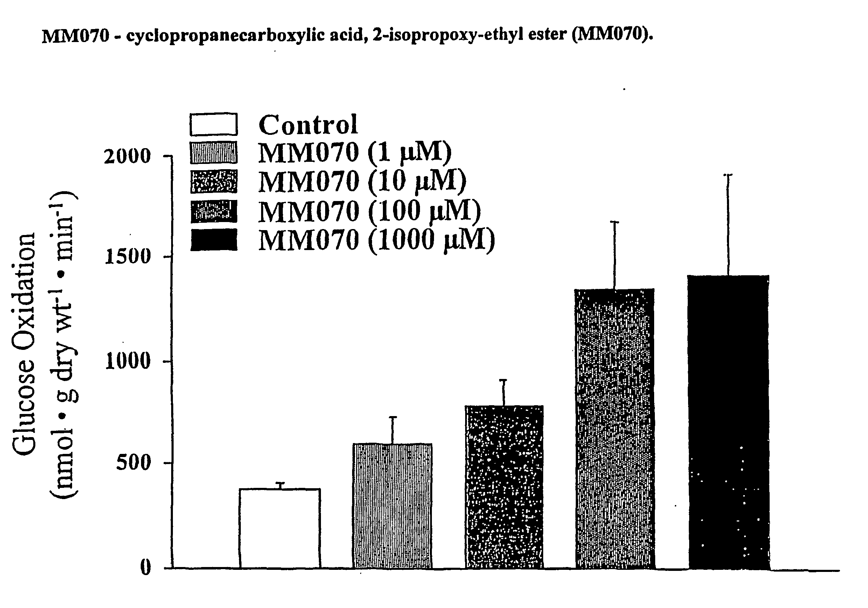

The procedure of Example A was followed except that cyclopropanecarboxylic acid, 2-isopropoxy-ethyl ester in 1 µM, 10 µM, 100 µM and 1000 µM amounts was added to the buffer in place of the cyclopropanecarboxylic acid, 2- [2- (2-methoxy-ethoxy)-ethoxy]-ethyl ester. The results are illustrated in Figure 3 and TABLE 2.

### (3) Glucose oxidation stimulation in myocardium cells treated with various cyclopropanecarboxylic acid and cyclobutanecarboxylic acid derivatives.

The procedure of Example A was followed except that various cyclobutanecarboxylic acid derivatives, cyclopropanecarboxylic acid derivatives and cyclobutanecarboxylic acid in the amounts of 100 µM or 1000 µM was added to the buffer in place of the cyclopropanecarboxylic acid, 2-[2-(2- methoxy-ethoxy)-ethoxy]-ethyl ester. The results are illustrated in TABLE 2.

### (4) Glucose oxidation stimulation in myocardium cells treated with cyclopropanecarboxylic acid.

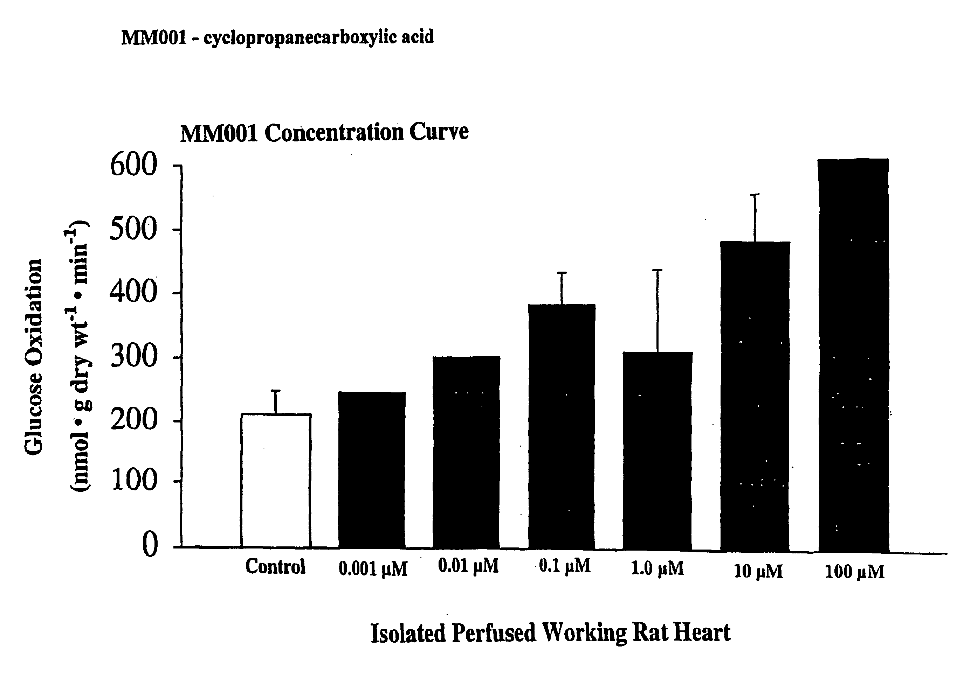

The procedure of Example A was followed except that cyclobutanecarboxylic acid in the amounts of 0.001 µM, 0.01 X µM, 0.1 µM, 1 µM, 10 µM, and 100 µM was added to the buffer X in place of the cyclopropanecarboxylic acid, 2-[2-(2-methoxy-ethoxy)-ethoxy]-ethyl ester. The results are illustrated in Figure 4 and TABLE 2.

**TABLE 2.**

| Compound of Example NO. | Compound | Screening Concentration (µM) | Glucose Oxidation (% above control) |
|---|---|---|---|
| 1 MM054 | 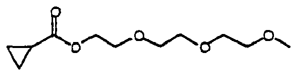 | 100 | 102% |
| | Cyclopropanecarboxylic acid 2-[2-(2-methoxy-ethoxy)-ethoxy]-ethyl ester | | |
| 2 MM055 | 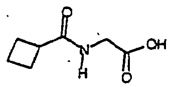 | 1000 µM | 58% |
| | (Cyclobutanecarbonyl-amino)-acetic acid | | |
| 3 MM056. | 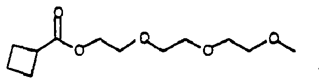 | 100 µM | 54% |
| | Cyclobutanecarboxylic acid 2-[2-(2-methoxythoxy)-ethoxy]-ethyl ester | | |
| 4 MM057 | 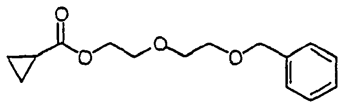 | 100 µM | 104% |
| | Cyclopropanecarboxylic acid 2-(2-banzylaxy-ethoxy)-ethyl ester | | |
| 5 MM058 | 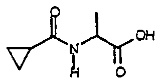 | 1000 µM | 40% |
| | 2-(Cyclopropanecarbonyl-amino)-propionic acid | | |
| 6 MM059 | 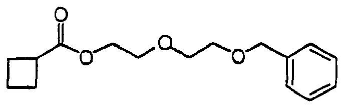 | 100 µM | 68% |
| | Cyclobutanecarboxylic acid 2-(2-benzyloxy-ethoxy)-ethyl ester | | |
| 7 MM060 | 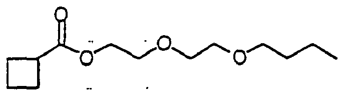 | 100 µM | 65% |
| | Cyclobutanecarboxylic acid, 2-(2-butoxy-ethoxy)-ethyl ester | | |
| 8 MM061 | 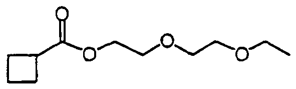 | Not screened | |
| | Cyclobutanecarboxylic acid, 2-(2-ethoxy-ethoxy)-ethyl ester | | |
| 9 MM062 | 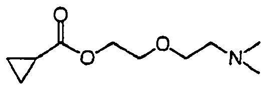 | 100 µM | 77% |
| | Cyclopropanecarboxylic acid 2-(2-dimethylamino-ethoxy)-ethyl ester | | |
| 10 MM063 | 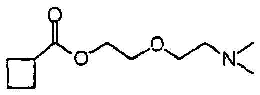 | 100 µM | 41% |
| | Cyclobutanecarboxylic acid 2-(2-dimethylaminothoxy)-ethyl ester | | |
| 11 MM064 | 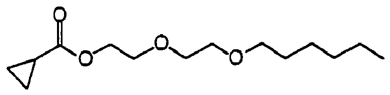 | 100 µM | 83% |
| | Cyclopropanecarboxylic acid 2-(2-hexyloxy-ethoxy)-ethyl ester | | |
| 12 MM065 | 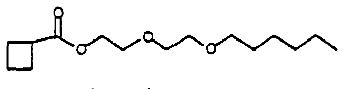 | 100 µM | 0% |
| | Cyclobutanecarboxylic acid 2-(2-hexyloxy-ethoxy)-ethyl ester | | |
| 13 MM066 | 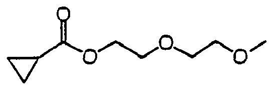 | 100 µM | 20% |
| | Cyclopropanecarboxylic acid 2-(2-methoxy-ethoxy)-ethyl ester | | |
| 14 MM067 | 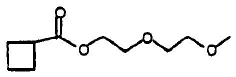 | 100 µM | 50% |
| | Cyclobutanecarboxylic acid 2-(2-methoxy-ethoxy)-ethyl ester | | |
| 15 MM068 | 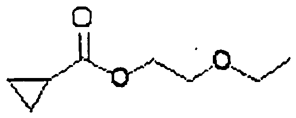 | 100 µM | 416% |
| | cyclopropanecarboxylic acid 2-ethoxy-ethyl ester | | |
| 16 MM069 | 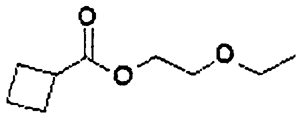 | 100 µM | 162% |
| | Cyclobutanecarboxylic acid 2-ethoxy-ethyl ester | | |
| 17 MM070 | 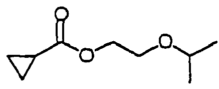 | 100 µM | 208% |
| | Cyclopropanecarboxylic acid 2-isopropoxy-ethyl ester | | |
| 18 MM071 | 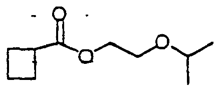 | 100 µM | 97% |
| | Cyclobutanecarboxylic acid 2-isopropoxy-ethyl ester | | |
| 19 MM072 | 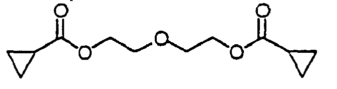 | 100 µM | 97% |
| | Cyclopropanecarboxylic acid, 2-(2-cyclopropanecarbonyloxy-ethoxy)-ethyl ester | | |
| 20 MM073 | 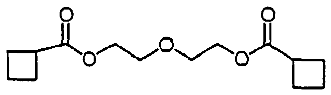 | 100 µM | 243% |
| | Cyclobutanecarboxylic acid, 2-(2-cyclobutanecarbonyloxy-ethoxy)-ethyl ester | | |
| 21 MM074 | 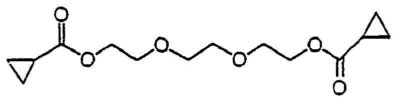 | 100 µM | 228% |
| | Cyclopropanecarboxylic acid,2-[2-(2-cyclopropanecarboxy-ethoxy)-ethoxy]-ethoxy]-ethyl ester | | |
| 22 MM075 | 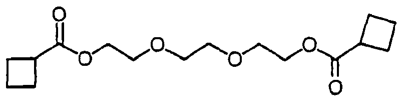 | 100 µM | 184% |
| | Cyclobutanecarboxylic acid,2-[2-(2-cyclobutanecarbonyloxy-ethoxy)-ethoxy]-ethyl ester | | |
| 23 MM076 | 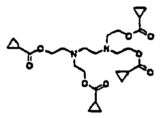 | 100 µM | 274% |
| | Cyclopropanecarboxylic acid 2-[{2-[bis(2-cyclopropanecarbonyloxy-ethyl)-amino]-ethyl}-(2-cyclopropanecarbonyloxy-ethyl)-amino] ethyl ester | | |
| 24 MM077 | 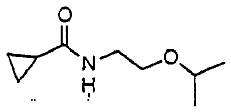 | 100 µM | 217% |
| | Cyclopropanecarboxylic acid (2-isopropoxy-ethyl)-amide | | |
| 25 MM078 | 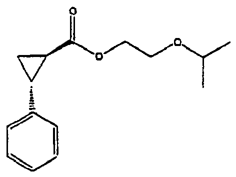 | 100 µM | 200% |
| | trans-2-Phenyl-cyclopropanecarboxylic acid 2-isopropoxy-ethyl ester | | |
| 26 MM079 | 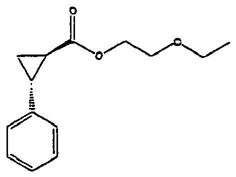 | not screened | |
| | trans-2-Phenyl-cyclopropanecarboxylic acid 2-ethoxy-ethyl ester | | |
| 27 MM080 | 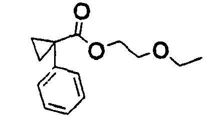 | not screened | |
| | 1-Phenyl-cyclopropanecarboxylic acid 2-ethoxy-ethyl ester | | |

## Claims

1. A compound selected from
cyclopropanecarboxylic acid, 2-[2-(2-methoxy-ethoxy)-ethoxy]-ethyl ester;
cyclobutanecarboxylic acid, 2-[2-(2-methoxy-ethoxy)-ethoxy]-ethyl ester;
cyclopropanecarboxylic acid, 2-(2-benzyloxy-ethoxy)-ethyl ester;
cyclobutanecarboxylic acid, 2-(2-benzyloxy-ethoxy)-ethyl ester;
cyclobutanecarboxylic acid, 2-(2-butoxy-ethoxy)-ethyl ester;
cyclobutanecarboxylic acid, 2-(2-ethoxy-ethoxy)-ethyl ester;
cyclopropanecarboxylic acid, 2-(2-dimethylamino-ethoxy)-ethyl ester;
cyclobutanecarboxylic acid, 2-(2-dimethylamino-ethoxy)-ethyl ester;
cyclopropanecarboxylic acid, 2-(2-hexyloxy-ethoxy)-ethyl ester;
cyclobutanecarboxylic acid, 2-(2-hexyloxy-ethoxy)-ethyl ester;
cyclopropanecarboxylic acid, 2-(2-methoxy-ethoxy)-ethyl ester;
cyclobutanecarboxylic acid, 2-(2-methoxy-ethoxy)-ethyl ester;
cyclopropanecarboxylic acid, 2-ethoxy-ethyl ester;
cyclobutanecarboxylic acid, 2-ethoxy-ethyl ester;
cyclopropanecarboxylic acid, 2-isopropoxy-ethyl ester; and
cyclobutanecarboxylic acid, 2-isopropoxy-ethyl ester;
or a pharmaceutically acceptable salt thereof.

2. A pharmaceutical composition comprising at least one compound of claim 1 or a pharmaceutically acceptable salt thereof;
and a pharmaceutically acceptable carrier, diluent, excipient or mixtures thereof.

3. The pharmaceutical composition according to claim 2, wherein said composition is in the form of tablets, pills, capsules, aqueous solutions, or sterile suspensions or solutions.

4. The compound as defined in claim 1 or a pharmaceutically acceptable salt thereof or the composition as defined in claim 2 or 3 for use in increasing glucose utilization in a cell, tissue or organ of a warm blooded animal.

5. Use of at least one compound as defined in claim 1 or a pharmaceutically acceptable salt thereof for the manufacture of a medicament for increasing glucose utilization in a cell, tissue or organ of a warm blooded animal.

6. The compound for use in increasing glucose utilization in a cell, tissue or organ of a warm blooded animal according to claim 4, wherein said organ is heart.

7. The use according to claim 5, wherein said organ is heart.

8. The compound for use in increasing glucose utilization in a cell, tissue or organ of a warm blooded animal according to claim 4, wherein said cell is a myocardial cell.

9. The use according to claim 5, wherein said cell is a myocardial cell.

10. The compound as defined in claim 1 or a pharmaceutically acceptable salt thereof or the composition as defined in claim 2 or 3 for use in the treatment of physiological conditions or disorders treatable by increasing glucose utilization.

11. Use of at least one compound as defined in claim 1 or a pharmaceutically acceptable salt thereof for the manufacture of a medicament for the treatment of physiological conditions or disorders treatable by increasing glucose utilization.

12. The compound for use in the treatment of physiological conditions or disorders treatable by increasing glucose utilization according to claim 10, wherein said disorder or condition is ischemic/reperfusion injury, post myocardial infarction, angina, heart failure, a cardiomyopathy, peripheral vascular disease, diabetes, and lactic acidosis or symptoms or side effects associated with open heart surgery, bypass surgery or heart transplant.

13. The use according to claim 11, wherein said disorder or condition is ischemic/reperfusion injury, post myocardial infarction, angina, heart failure, a cardiomyopathy, peripheral vascular disease, diabetes, and lactic acidosis or symptoms or side effects associated with open heart surgery, bypass surgery or heart transplant.

14. The compound for use in the treatment of physiological conditions or disorders treatable by increasing glucose utilization according to claim 10, wherein said disorder or condition is ischemic/reperfusion injury.

15. The use according to claim 11, wherein said disorder or condition is ischemic/reperfusion injury.

16. A kit containing a pharmaceutical composition as defined in claim 2 or 3.

17. A kit according to claim 16, wherein said kit comprises a label or packaging insert containing instructions for use, in vitro, in vivo or ex vivo, of components of said kit.

## Patentansprüche

1. Eine Verbindung, ausgewählt aus
Cyclopropancarbonsäure, 2-[2-(2-methoxy-ethoxy)-ethoxy]-ethylester;
Cyclobutancarbonsäure, 2-[2-(2-methoxy-ethoxy)-ethoxy]-ethylester;
Cyclopropancarbonsäure, 2-(2-benzyloxy-ethoxy)-ethylester;
Cyclobutancarbonsäure, 2-(2-benzyloxy-ethoxy)-ethylester;
Cyclobutancarbonsäure, 2-(2-butoxy-ethoxy)-ethylester;
Cyclobutancarbonsäure, 2-(2-ethoxy-ethoxy)-ethylester;
Cyclopropancarbonsäure, 2-(2-dimethylamino-ethoxy)-ethylester;
Cyclobutancarbonsäure, 2-(2-dimethylamino-ethoxy)-ethylester;
Cyclopropancarbonsäure, 2-(2-hexyloxy-ethoxy)-ethylester;
Cyclobutancarbonsäure, 2-(2-hexyloxy-ethoxy)-ethylester;
Cyclopropancarbonsäure, 2-(2-methoxy-ethoxy)-ethylester;
Cyclobutancarbonsäure, 2-(2-methoxy-ethoxy)-ethylester;
Cyclopropancarbonsäure, 2-ethoxy-ethylester;
Cyclobutancarbonsäure, 2-ethoxy-ethylester;
Cyclopropancarbonsäure, 2-isopropoxy-ethylester; und
Cyclobutancarbonsäure, 2-isopropoxy-ethylester;
oder ein pharmazeutisch verträgliches Salz davon.

2. Eine pharmazeutische Zusammensetzung, umfassend mindestens eine Verbindung nach Anspruch 1 oder ein pharmazeutisch verträgliches Salz davon;
und ein(en) pharmazeutisch verträglichen/s Träger, Verdünnungsmittel, Exzipienten oder Gemische davon.

3. Die pharmazeutische Zusammensetzung gemäß Anspruch 2, wobei die Zusammensetzung in der Form von Tabletten, Pillen, Kapseln, wässrigen Lösungen oder sterilen Suspensionen oder Lösungen vorliegt.

4. Die Verbindung wie in Anspruch 1 definiert oder ein pharmazeutisch verträgliches Salz davon oder die Zusammensetzung wie in Anspruch 2 oder 3 definiert zur Verwendung zur Erhöhung der Glucosenutzung in einer/m Zelle, Gewebe oder Organ eines Warmblüters.

5. Verwendung mindestens einer Verbindung wie in Anspruch 1 definiert oder eines pharmazeutisch verträglichen Salzes davon zur Herstellung eines Medikaments zur Erhöhung der Glucosenutzung in einer/m Zelle, Gewebe oder Organ eines Warmblüters.

6. Die Verbindung zur Verwendung zur Erhöhung der Glucosenutzung in einer/m Zelle, Gewebe oder Organ eines Warmblüters gemäß Anspruch 4, wobei das Organ ein Herz ist.

7. Die Verwendung gemäß Anspruch 5, wobei das Organ ein Herz ist.

8. Die Verbindung zur Verwendung zur Erhöhung der Glucosenutzung in einer/m Zelle, Gewebe oder Organ eines Warmblüters gemäß Anspruch 4, wobei die Zelle eine Myokardzelle ist.

9. Die Verwendung gemäß Anspruch 5, wobei die Zelle eine Myokardzelle ist.

10. Die Verbindung wie in Anspruch 1 definiert oder ein pharmazeutisch verträgliches Salz davon oder die Zusammensetzung wie in Anspruch 2 oder 3 definiert zur Verwendung bei der Behandlung von physiologischen Zuständen oder Störungen, die durch Erhöhung der Glukosenutzung behandelt werden können.

11. Verwendung mindestens einer Verbindung wie in Anspruch 1 definiert oder eines pharmazeutisch verträglichen Salzes davon zur Herstellung eines Medikaments zur Behandlung von physiologischen Zuständen oder Störungen, die durch Erhöhung der Glukosenutzung behandelt werden können.

12. Die Verbindung zur Verwendung bei der Behandlung von physiologischen Zuständen oder Störungen, die durch Erhöhung der Glukosenutzung behandelt werden können, gemäß Anspruch 10, wobei die Störung oder der Zustand Ischämie-/Reperfusion-Schaden, Postmyokardinfakt, Angina, Herzversagen, eine Kardiomyopathie, periphere Gefäßerkrankung, Diabetes und Laktatazidose oder Symptome oder Nebenwirkungen im Zusammenhang mit Operation am offenen Herzen, Bypassoperation oder Herztransplantation ist.

13. Die Verwendung gemäß Anspruch 11, wobei die Störung oder der Zustand Ischämie/Reperfusion-Schaden, Postmyokardinfakt, Angina, Herzversagen, eine Kardiomyopathie, periphere Gefäßerkrankung, Diabetes und Laktatazidose oder Symptome oder Nebenwirkungen im Zusammenhang mit Operation am offenen Herzen, Bypassoperation oder Herztransplantation ist.

14. Die Verbindung zur Verwendung bei der Behandlung von physiologischen Zuständen oder Störungen, die durch Erhöhung der Glukosenutzung behandelt werden können, gemäß Anspruch 10, wobei die Störung oder der Zustand Ischämie-/Reperfusion-Schaden ist.

15. Die Verwendung gemäß Anspruch 11, wobei die Störung oder der Zustand Ischämie/Reperfusion-Schaden ist.

16. Ein Kit, enthaltend eine pharmazeutische Zusammensetzung wie in Anspruch 2 oder 3 definiert.

17. Ein Kit gemäß Anspruch 16, wobei das Kit ein Etikett oder eine Packungsbeilage umfasst, enthaltend Instruktionen zur Verwendung, in vitro, in vivo oder ex vivo, der Komponenten des Kits.

## Revendications

1. Composé choisi parmi
l'ester 2-[2-(2-méthoxy-éthoxy)-éthoxy]-éthylique d'acide cyclopropane carboxylique ;
l'ester 2-[2-(2-méthoxy-éthoxy)-éthoxy]-éthylique d'acide cyclobutane carboxylique ;
l'ester 2-(2-benzyloxy-éthoxy)-éthylique d'acide cyclopropane carboxylique ;
l'ester 2-(2-benzyloxy-éthoxy)-éthylique d'acide cyclobutane carboxylique ;
l'ester 2-(2-butoxy-éthoxy)-éthylique d'acide cyclobutane carboxylique ;
l'ester 2-(2-éthoxy-éthoxy)-éthylique d'acide cyclobutane carboxylique ;
l'ester 2-(2-diméthylamino-éthoxy)-éthylique d'acide cyclopropane carboxylique ;
l'ester 2-(2-diméthylamino-éthoxy)-éthylique d'acide cyclobutane carboxylique ;
l'ester 2-(2-hexyloxy-éthoxy)-éthylique d'acide cyclopropane carboxylique ;
l'ester 2-(2-hexyloxy-éthoxy)-éthylique d'acide cyclobutane carboxylique ;
l'ester 2-(2-méthoxy-éthoxy)-éthylique d'acide cyclopropane carboxylique ;
l'ester 2-(2-méthoxy-éthoxy)-éthylique d'acide cyclobutane carboxylique ;
l'ester 2-éthoxy-éthylique d'acide cyclopropane carboxylique ;
l'ester 2-éthoxy-éthylique d'acide cyclobutane carboxylique ;
l'ester 2-isopropoxy-éthylique d'acide cyclopropane carboxylique ; et
l'ester 2-isopropoxy-éthylique d'acide cyclobutane carboxylique ;
ou un sel pharmaceutiquement acceptable de celui-ci.

2. Composition pharmaceutique comprenant au moins un composé selon la revendication 1 ou un sel pharmaceutiquement acceptable de celui-ci ;
et un support, un diluant ou un excipient pharmaceutiquement acceptables ou mélanges de ceux-ci.

3. Composition pharmaceutique selon la revendication 2, ladite composition se présentant sous la forme de comprimés, de pilules, de capsules, de solutions aqueuses, ou de suspensions ou de solutions stériles.

4. Composé selon la revendication 1 ou sel pharmaceutiquement acceptable de celui-ci ou composition selon la revendication 2 ou 3, destiné(e) à stimuler l'utilisation de glucose dans une cellule, un tissu ou un organe d'un animal à sang chaud.

5. Utilisation d'au moins un composé selon la revendication 1 ou d'un sel pharmaceutiquement acceptable de celui-ci pour la fabrication d'un médicament destiné à stimuler l'utilisation de glucose dans une cellule, un tissu ou un organe d'un animal à sang chaud.

6. Composé destiné à stimuler l'utilisation de glucose dans une cellule, un tissu ou un organe d'un animal à sang chaud selon la revendication 4, dans lequel ledit organe est le coeur.

7. Utilisation selon la revendication 5, dans laquelle ledit organe est le coeur.

8. Composé destiné à stimuler l'utilisation de glucose dans une cellule, un tissu ou un organe d'un animal à sang chaud selon la revendication 4, dans lequel ladite cellule est une cellule myocardique.

9. Utilisation selon la revendication 5, dans laquelle ladite cellule est une cellule myocardique.

10. Composé selon la revendication 1 ou sel pharmaceutiquement acceptable de celui-ci ou composition selon la revendication 2 ou 3, destiné(e) au traitement de conditions ou de troubles physiologiques pouvant être traités en stimulant l'utilisation de glucose.

11. Utilisation d'au moins un composé selon la revendication 1 ou d'un sel pharmaceutiquement acceptable de celui-ci pour la fabrication d'un médicament destiné au traitement de conditions ou de troubles physiologiques pouvant être traités en stimulant l'utilisation de glucose.

12. Composé destiné au traitement de conditions ou de troubles physiologiques pouvant être traités en stimulant l'utilisation de glucose selon la revendication 10, dans lequel ledit trouble ou ladite condition est une lésion d'ischémie-reperfusion, un infarctus du myocarde, une angine, une insuffisance cardiaque, une cardiomyopathie, un acrosyndrome, un diabète et une acidose lactique ou des symptômes ou des effets secondaires associés à une chirurgie à coeur ouvert, une opération de pontage ou une transplantation cardiaque.

13. Utilisation selon la revendication 11, dans laquelle ledit trouble ou ladite condition est une lésion d'ischémie-reperfusion, un infarctus du myocarde, une angine, une insuffisance cardiaque, une cardiomyopathie, un acrosyndrome, un diabète et une acidose lactique ou des symptômes ou des effets secondaires associés à une chirurgie à coeur ouvert, une opération de pontage ou une transplantation cardiaque.

14. Composé destiné au traitement de conditions ou de troubles physiologiques pouvant être traités en stimulant l'utilisation de glucose selon la revendication 10, dans lequel ledit trouble ou ladite condition est une lésion d'ischémie-reperfusion.

15. Utilisation selon la revendication 11, dans laquelle ledit trouble ou ladite condition est une lésion d'ischémie-reperfusion.

16. Trousse contenant une composition pharmaceutique selon la revendication 2 ou 3.

17. Trousse selon la revendication 16, ladite trousse comprenant une étiquette ou une notice d'emballage comportant des instructions d'utilisation, in vitro, in vivo ou ex vivo, pour les composants de ladite trousse.
